(19) Europäisches Patentamt
European Patent Office
Office européen des brevets

(11) EP 3 758 510 B1

(12) **EUROPEAN PATENT SPECIFICATION**

(45) Date of publication and mention of the grant of the patent:
**26.08.2026 Bulletin 2026/35**

(21) Application number: **19760572.8**

(22) Date of filing: **27.02.2019**

(51) International Patent Classification (IPC):
***A23L 29/25*** *(2016.01)* ***C08B 37/00*** *(2006.01)*
***A61K 49/04*** *(2006.01)* ***A61K 47/36*** *(2006.01)*

(52) Cooperative Patent Classification (CPC):
**A23L 29/25; A23L 2/52; A23L 29/238; A23L 33/40; C08B 37/00; C08B 37/0003; C08L 5/00**

(86) International application number:
**PCT/AU2019/050171**

(87) International publication number:
**WO 2019/165506 (06.09.2019 Gazette 2019/36)**

(54) **AN INGREDIENT**

BESTANDTEIL

INGRÉDIENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.02.2018 AU 2018900635**

(43) Date of publication of application:
**06.01.2021 Bulletin 2021/01**

(73) Proprietor: **Trisco ICAP Pty Ltd**
**Brisbane, Queensland 4000 (AU)**

(72) Inventors:
- **TRISTRAM, Michael**
  **Brisbane, Queensland 4000 (AU)**
- **MOSSEL, Brenda**
  **Brisbane, Queensland 4000 (AU)**
- **SKARSHEWSKI, Peter**
  **Brisbane, Queensland 4000 (AU)**

(74) Representative: **HGF**
**HGF Limited**
**4th Floor, 1 City Square**
**Leeds LS1 2ES (GB)**

(56) References cited:
**WO-A1-02/069981**
**WO-A1-2007/009217**
**WO-A1-2011/140598**
**WO-A1-2013/087916**
**WO-A2-03/045308**
**WO-A2-2006/053761**
**AU-A4- 2017 101 619**
**CN-A- 102 558 377**
**JP-A- 2001 048 810**
**JP-A- 2012 200 190**

- **TROFIMOVA, N.N. ET AL.: "Polysaccharides from Larch Biomass", THE COMPLEX WORLD OF POLYSACCHARIDES, 31 October 2012 (2012-10-31), XP055634271, DOI: 10.5772/53809**
- **CICHERO, J.A.Y: "Thickening agents used for dysphagia management: effect on bioavailability of water, medication and feelings of satiety", NUTRITION JOURNAL, vol. 12, 2013, pages 54, XP021151069, doi:10.1186/1475-2891-12-54**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 758 510 B1

**Description**

FIELD OF THE INVENTION

This invention relates to a polysaccharide-based ingredient for use in preparing a food thickening composition and method of making same. The invention further relates to a stable liquid composition containing the polysaccharide-based ingredient and a thickening agent for increasing the viscosity of a liquid or semi-liquid foodstuff and a method of using same, according to the appended claims.

BACKGROUND TO THE INVENTION

It is often desirable to provide viscous thickened liquids, particularly for the geriatric and convalescent markets. The thickened liquids need to be of a particular, known and repeatable viscosity to be applicable to these markets.

Predetermined liquid viscosities have been developed by a number of regulatory bodies that are considered to have a clinically significant benefit in 'slowing down' a dysphagia patient's swallow so that common co-morbidities of the disorder, such as aspiration pneumonia, are prevented. In light of the varying severity of swallowing disorders, the following professional guidelines are generally practiced clinically: mildly thick (nectar consistency); moderately thick (honey consistency); and thick (pudding consistency). These guidelines typically correlate to 150, 400 and 900 mPa.s, respectively.

Thickening beverages for the management of dysphagia in institutions and homes is typically achieved using powdered thickeners, which have been "instantised' through physical modification such as agglomeration. Such powders, however, can have limitations, such as the inability to deliver an accurate volumetric dose to a foodstuff as required and the requirement for specialised mixing equipment in order to obtain sufficient shear forces to ensure adequate dispersion thereof. Moreover, the time taken for powdered thickeners to express their viscosity is typically not instantaneous (i.e., <30 secs), but rather may take up to several minutes to bring a foodstuff to its maximal or desired viscosity. Commercially available liquid thickening agents that function by way of expressing the viscosity of the thickening agent in a concentrated solution and diluting back to a desired concentration, are similarly limited by the amount of shear required to disperse and express their viscosity. Such liquid thickening agents may also not be stable for sufficient periods of time when stored at room temperature, resulting in the separation of one or more components thereof.

WO2006053761 describes plant gums that have been subjected to hydrolysis to degrade a portion of the protein and/or the polysaccharide thereof, and their use in compositions containing fat-soluble active ingredients (e.g., vitamins) and/or colourants in a matrix based on modified plant gums. Such compositions are disclosed as being suitable for the enrichment, fortification and/or coloration of food, beverages, animal feed, cosmetics and pharmaceutical compositions.

Thus, there remains a need for a stable liquid thickener composition that may be used, for example, to feed subjects suffering from a mastication and/or deglutition disorder, such as dysphagia, which overcomes one or more of the inherent limitations of commercially available liquid and/or powdered thickener compositions.

SUMMARY OF THE INVENTION

In a first aspect, the invention provides a polysaccharide-based ingredient for use in preparing a food thickening composition comprising:

a polysaccharide-based source material selected from the group consisting of a *Larix occidentalis* polysaccharide extract, a *Larix laricina* polysaccharide extract, an Acacia tree polysaccharide extract, a *Larix decidua* polysaccharide extract, a *Larix sibirica* polysaccharide extract and any combination thereof;
wherein the polysaccharide-based source material having been subjected to a protein hydrolysis step, wherein the hydrolysis step includes:

(a) a heat treatment step carried out at a temperature of from about 55°C to about 90° C; and
(b) an acid treatment step carried out at a pH of about 3 to about 5.

In some embodiments, the polysaccharide-based has a protein content that is less than 2 wt% or suitably less than 0.1wt%, and is capable of modulating and/or controlling the water binding ability of a thickening agent.

In some embodiments, the protein hydrolysis step having lowered an initial protein level of the polysaccharide-based source material to a second protein level.

In one embodiment, the polysaccharide-based source material has further been subjected to a protein extraction step.

In a second aspect, the invention provides a method of preparing a polysaccharide-based ingredient for use in

preparing a food thickening composition including the steps of:

(i) providing a polysaccharide-based source material selected from the group consisting of a *Larix occidentalis* polysaccharide extract, a *Larix laricina* polysaccharide extract, an Acacia tree polysaccharide extract, a *Larix decidua* polysaccharide extract, a *Larix sibirica* polysaccharide extract and any combination thereof; and
(ii) hydrolysing a portion of protein of the polysaccharide-based source material wherein the hydrolysis step includes:

(a) a heat treatment step carried out at a temperature of from about 55°C to about 90° C; and
(b) an acid treatment step carried out at a pH of about 3 to about 5;

to thereby prepare the polysaccharide-based ingredient.

In some embodiments, the polysaccharide-based has a protein content that is less than 2 wt% or suitably less than 0.1wt%, and is capable of modulating and/or controlling the water binding ability of a thickening agent. In some embodiments, step (ii) lowers an initial protein level of the polysaccharide-based source material to a second protein level.

In one embodiment, the method of the present aspect further includes the step of extracting a portion of hydrolysed protein from the polysaccharide-based source material of (ii).

With respect to the aforementioned aspects, the protein hydrolysis step suitably comprises one or more of protease treatment, alkali treatment, microwave radiation treatment, and metal aqua ion treatment.

In particular embodiments of the first and second aspects, acid treatment includes contacting the polysaccharide-based source material with a food grade acid selected from the group consisting of lactic acid, phosphoric acid, citric acid, malic acid, ascorbic acid, formic acid, fumaric acid, succinic acid, tartaric acid, gluconic acid and any combination thereof. Preferably, the food grade acid is or comprises gluconic acid, such as that derived at least in part from glucono delta-lactone.

Referring to the above aspects, acid treatment is suitably carried out at a pH of about 3 to about 5. Preferably, acid treatment is carried out at a pH of about 4.0 to 4.5 and more preferably at a pH of about 4.2 to 4.4. In an embodiment, the acid treatment step is carried out at a pH of about 3.9 to 4.4.

In certain embodiments of the first and second aspects, heat treatment is carried out at a temperature of from about 55°C to about 90° C. More preferably, heat treatment is carried out at a temperature of from about 65°C to about 85°C and even more preferably from about 70°C to about 80°C.

Suitably, the protein extraction step of the first and second aspects includes one or more of gravity separation, centrifugation, size exclusion chromatography, hydrophobic interaction chromatography, ion exchange chromatography, free flow electrophoresis, metal binding, immunoaffinity chromatography and immunoprecipitation.

In relation to the first and second aspects, the protein hydrolysis step is preferably carried out for a period of time from about 15 minutes hours to about 30 hours, more preferably from about 8 hours to about 20 hours and even more preferably from about 30 minutes to about 2 hours.

In a third aspect, the invention provides a stable liquid composition having a viscosity of less than 4000 cP measured by a rheometer, comprising:

(i) one or a plurality of thickening agents wherein the thickening agent is selected from the group consisting of agar, alginic acid, carrageenan, guar gum, gum tragacanth, gum ghatti, microcrystalline cellulose, sodium carboxymethylcellulose, methyl cellulose, hydroxypropylmethylcellulose, hydroxypropylcellulose, methylethylcellulose, gum karaya, xanthan gum, locust bean gum, tara gum, psyllium seed gum, quince seed gum, a pectin, furcellaran, gellan gum, konjac, sodium alginate and any combination thereof and wherein the thickening agent is present in an amount from about 3% to about 30% by weight of the stable liquid composition; and
(ii) a polysaccharide-based ingredient comprising a polysaccharide-based source material selected from the group consisting of a *Larix occidentalis* polysaccharide extract, a *Larix laricina* polysaccharide extract, an Acacia tree polysaccharide extract, a *Larix decidua* polysaccharide extract, a *Larix sibirica* polysaccharide extract and any combination thereof, wherein the polysaccharide-based source material has been subjected to a protein hydrolysis step;

wherein addition of the composition to an aqueous liquid or aqueous liquid-solid mixture foodstuff increases the viscosity of said foodstuff.

In an embodiment, the protein hydrolysis step comprises one or more of heat treatment, protease treatment, acid treatment, alkali treatment, microwave radiation treatment, and metal aqua ion treatment.

In one embodiment, the composition has a viscosity of less than 2000 cP.

In particular embodiments, the composition has a water activity of greater than 95%.

In one preferred embodiment, the composition is stable for at least six months at room temperature.

Suitably, the composition is configured to produce substantially no change in an impedance level of the foodstuff

when added thereto. In this regard, the foodstuff preferably is or comprises a medium for use in determining a diagnosis and/or a prognosis of dysphagia.

In a fourth aspect, the invention provides a method for increasing the viscosity of an aqueous liquid or aqueous liquid solid mixture foodstuff, the method including the steps of:

(a) adding to the foodstuff a stable liquid composition of the third aspect; and
(b) mixing the foodstuff and the composition so as to promote increasing the viscosity of said foodstuff by the composition.

Suitably, the mixing step includes applying low-shear mixing. To this end, the low-shear mixing is preferably applied for about 30 seconds or less to achieve a maximal viscosity of the foodstuff. More preferably, the low-shear mixing is applied for about 10 to about 30 seconds to achieve a maximal viscosity of the foodstuff. In particular embodiments, the low-shear mixing comprises stirring said composition at a speed of from about 10 rpm to about 40 rpm.

In certain embodiments, the viscosity of the foodstuff is suitably increased to greater than 95 cP.

In referring to the above aspect, the foodstuff of increased viscosity is suitably for feeding a subject suffering from a mastication and/or deglutition disease, disorder or condition. Preferably, the mastication and/or deglutition disease, disorder or condition is or comprises dysphagia.

In a fifth aspect, the invention provides a method of producing a stable liquid composition, including the steps of:

(i) providing a polysaccharide-based ingredient comprising a polysaccharide-based source material selected from the group consisting of a *Larix occidentalis* polysaccharide extract, a *Larix laricina* polysaccharide extract, an Acacia tree polysaccharide extract, a *Larix decidua* polysaccharide extract, a *Larix sibirica* polysaccharide extract and any combination thereof, wherein the polysaccharide-based source material has been subjected to a protein hydrolysis step;
(ii) adding one or a plurality of thickening agents to the polysaccharide-based ingredient wherein the thickening agent is selected from the group consisting of agar, alginic acid, carrageenan, guar gum, gum tragacanth, gum ghatti, microcrystalline cellulose, sodium carboxymethylcellulose, methyl cellulose, hydroxypropylmethylcellulose, hydroxypropylcellulose, methylethylcellulose, gum karaya, xanthan gum, locust bean gum, tara gum, psyllium seed gum, quince seed gum, a pectin, furcellaran, gellan gum, konjac, sodium alginate and any combination thereof and wherein the thickening agent is present in an amount from about 3% to about 30% by weight of the stable liquid composition; and
(iii) mixing the mixture of step (ii) to thereby produce the stable liquid composition.

Suitably, the stable liquid composition is that of the third aspect.

As used herein, except where the context requires otherwise, the term *"comprise"* and variations of the term, such as *"comprising", "comprises"* and *"comprised',* are not intended to exclude further elements, components, integers or steps but may include one or more unstated further elements, components, integers or steps.

It will be appreciated that the indefinite articles "a" and *"an"* are not to be read as singular indefinite articles or as otherwise excluding more than one or more than a single subject to which the indefinite article refers. For example, "a" polysaccharide includes one polysaccharide, one or more polysaccharides and a plurality of polysaccharides.

## BRIEF DESCRIPTION OF THE DRAWINGS

To assist in understanding the invention and to enable a person skilled in the art to put the invention into practical effect, preferred embodiments of the invention will be described by way of example only with reference to the accompanying drawings, in which:

FIG 1 provides an embodiment of a manufacturing process for a food thickening composition;
FIG 2 provides percentage reduction in retentate at each stage of the manufacturing process for a food thickening composition of FIG 1;
FIG 3 demonstrates 10 mL of a diagnostic bolus medium across four consistencies (unthickened, thickened to level 150, level 400 and level 900) thickened with various concentrations of a liquid composition containing an embodiment of the polysaccharide-based ingredient of the invention;
FIG 4 provides SDS PAGE of the samples taken during processing; PM indicates lanes containing SeeBlue Plus2 prestained protein ladder. Lane 1: first extract (TSC 1), lane 2: second extract (TSC 2), lane 3: bulk retentate 2 (TSC 3), lane 4: third extract (TSC 4), lane 5: bulk retentate 3 (TSC 5), lane 6: bulk retentate 4 (TSC 6), lane 7: FG-commercial product (TSC 7) and lane 8: bulk retentate 1 (TSC 8). The red arrows are indicating the protein bands at 60, 40 and 20 kDa (from top to bottom) which have been used for LC-MS analysis;

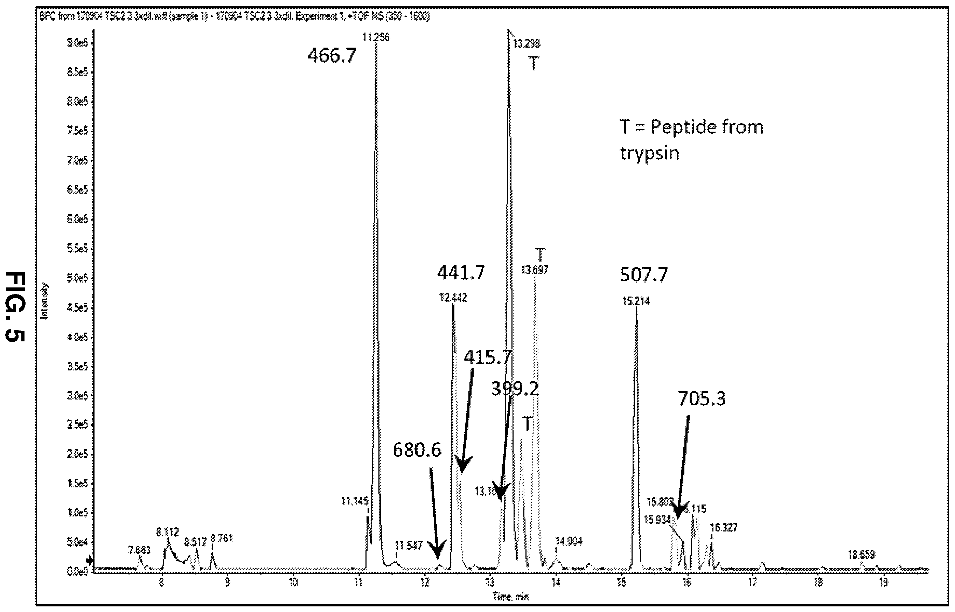

FIG 5 illustrates a base peak chromatogram of the sample TSC2-3 showing the m/z values for the abundant but unmatched peptide peaks. Autolytic peptides from trypsin itself are indicated by a T.

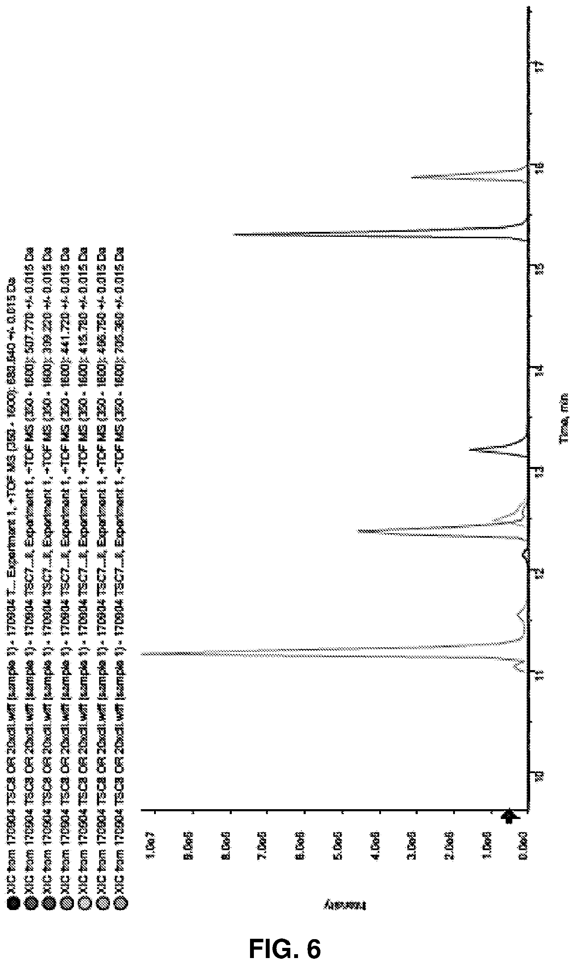

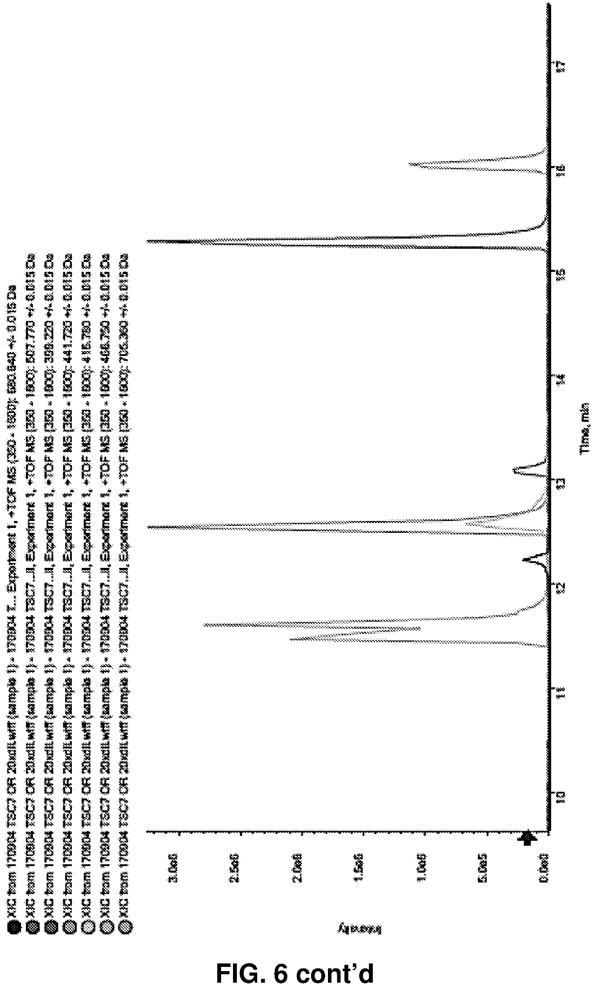

FIG 6 demonstrates extracted Ion chromatograms for seven abundant, unmatched peptides in the initial hydrocolloid (bulk retentate 1, Figure 5A) and the final product (bulk retentate 5, Figure 5B);

FIG 7 illustrates extracted ion chromatograms for gel bands from sample TSC 1; peptides for the bands at 60 kDa (TSC1-1), 40 kDa (TSC1-2) and 20 kDa (TSC1-3) are shown in figure 7A, 7B and 7C, respectively;

FIG 8 illustrates extracted ion chromatograms for gel bands from sample TSC 2; peptides for the bands at 60 kDa (TSC2-1), 40 kDa (TSC2-2) and 20 kDa (TSC2-3) are shown in figure 8A, 8B and 8C, respectively;

FIG 9 demonstrates extracted ion chromatograms for gel bands from sample TSC 3; peptides for the bands at 60 kDa (TSC3-1), 40 kDa (TSC3-2) and 20 kDa (TSC3-3) are shown in figure 9A, 9B and 9C, respectively;

FIG 10 demonstrates extracted ion chromatograms for gel bands from sample TSC 5; peptides for the bands at 40 kDa (TSC5-1) and 20 kDa (TSC5-2) are shown in figure 10A and 10B, respectively;

FIG 11 illustrates extracted ion chromatograms for gel bands from sample TSC 5; peptides for the bands at 40 kDa (TSC8-2) and 20 kDa (TSC8-3) are shown in figure 11A and 11B, respectively.

## DETAILED DESCRIPTION OF THE INVENTION

The invention advantageously provides a polysaccharide-based ingredient for use in preparing a liquid food thickening composition that is stable (e.g., for up to six months at room temperature) and can be control released and viscosity expressed when dispersed in liquid or semi-liquid foodstuffs. Foodstuffs thickened by such a liquid food thickening composition, such as electrolyte solutions, may also demonstrate utility in a diagnostic and/or prognostic setting owing to the ability of the composition to produce little or no change in an impedance level of the foodstuff when added thereto. The liquid food thickening composition comprising the polysaccharide-based ingredient also requires only the use of low shear mixing forces (e.g., gentle mixing with a spoon) when added to a foodstuff so as to rapidly express its viscosity therein (e.g., <30 secs).

In one aspect, the invention provides a polysaccharide-based ingredient as defined in the first aspect.

In some embodiments, the protein hydrolysis step having lowered an initial protein level of the polysaccharide-based source material to a second protein level.

In one embodiment, the polysaccharide-based source material has further been subjected to a protein extraction step.

In a related aspect, the invention provides a method of preparing a polysaccharide-based ingredient as defined in the second aspect.

In some embodiments, step (ii) lowers an initial protein level of the polysaccharide-based source material to a second protein level.

In one embodiment, the method of the present aspect further includes the step of extracting a portion of hydrolysed protein from the polysaccharide-based source material of (ii).

Accordingly, the polysaccharide-based ingredient refers to a modified polysaccharide-based source material, such as a plant gum, which has been subjected to hydrolysis to degrade a protein portion and, where appropriate or optionally, a polysaccharide portion thereof.

The term *"polysaccharide",* as used herein, generally refers to polymers formed from about 10 to over 100,000 saccharide units linked to each other by hemiacetal or glycosidic bonds. The polysaccharide may be either a straight chain, singly branched, or multiply branched wherein each branch may have additional secondary branches, and the monosaccharides may be standard D- or L-cyclic sugars in the pyranose (6-membered ring) or furanose (5- membered ring) forms such as D-fructose and D-galactose, respectively. Additionally, they may be cyclic sugar derivatives, deoxy sugars, sugar, sugar acids, or multi-derivatized sugars. As would be understood by the skilled artisan, polysaccharide preparations, and in particular those isolated from nature, typically comprise molecules that are heterogeneous in molecular weight.

The term "polysaccharide-based source material" refers to materials containing one or a plurality of polysaccharides as a major component thereof (e.g., the polysaccharide-based source material comprises at least about 20%, 25%, 30%, 35%, 40%, 45%, 50%, 55%, 60%, 65%, 70%, 75%, 80%, 85%, 90%, 95% or any range therein of polysaccharide by weight of the polysaccharide-based source material). Accordingly, the polysaccharide-based source material may include other components, such as protein, lipid etc, as a minor component thereof.

As described herein, the polysaccharide-based source material, such as a plant extract or gum described herein also contain a protein portion as a minor component thereof. In certain embodiments, the polysaccharide-based source material has an initial protein content or level of about or less than about 20 wt% (e.g., 20, 19, 18, 17, 16, 15, 14, 13, 12, 11, 10, 9, 8, 7, 6, 5, 4, 3, 2, 1, 0.5 wt% and any range therein), preferably less than about 10 wt% and more preferably less than about 6 wt% based on the total weight of the polysaccharide-based source material. As such, in some embodiments, the second protein content or level produced following treatment of the polysaccharide-based source material in step (ii)

above is less than about 20 wt% (e.g., 20, 19, 18, 17, 16, 15, 14, 13, 12, 11, 10, 9, 8, 7, 6, 5, 4, 3, 2, 1, 0.5 wt% and any range therein).

By "protein" is meant an amino acid polymer. The amino acids may be natural or non-natural amino acids, D- or L-amino acids as are well understood in the art. The term "protein" includes and encompasses "peptide", which is typically used to describe a protein having no more than fifty (50) amino acids and "polypeptide", which is typically used to describe a protein having more than fifty (50) amino acids.

By "protein hydrolysis" or "hydrolysing the protein" is meant a process of cleavage or breakage of the chemical bonds that hold the protein material together such that the protein is hydrolyzed or broken down into smaller peptides or protein fragments typically having a reduced molecular weight than the protein in its original (i.e., unhydrolyzed) state. In one embodiment, the method of the present invention partially hydrolyses the lignocellulosic material. "Partial hydrolysis" or "partially hydrolyses" and any grammatical variants thereof, as used herein, refer to the hydrolysis reaction cleaving or breaking less than 100% of the chemical bonds that hold the protein together. By way of example, protein can be hydrolyzed using heat treatment, an acid, a base, one or more enzymes, or any combination of any of these.

Accordingly, in particular embodiments, the protein hydrolysis step comprises one or more of protease treatment, alkali treatment, microwave radiation treatment and metal aqua ion treatment. The protein hydrolysis step may include: (a) sequentially with acid treatment and then heat treatment; or (b) sequentially with heat treatment and then acid treatment.

As used herein, "treating" or "treatment" may refer to, for example, contacting, soaking, steam impregnating, spraying, suspending, immersing, saturating, dipping, wetting, rinsing, washing, submerging, and/or any variation and/or combination thereof.

The term "protease" is defined herein as an enzyme that hydrolyses peptide bonds. The term "protease" can include any enzyme belonging to the EC 3.4 enzyme group (including each of the thirteen subclasses thereof). The EC number refers to Enzyme Nomenclature 1992 from NC-IUBMB, Academic Press, San Diego, California. As will be appreciated, proteases are classified on the basis of their catalytic mechanism into the following groups: Serine proteases (S), Cysteine proteases (C), Aspartic proteases (A), Metallo proteases (M), and Unknown, or as yet unclassified, proteases (U). (see, e.g., Handbook of Proteolytic Enzymes, A.J.Barrett, N.D.Rawlings, J.F.Woessner (eds), Academic Press (1998)),

The proteases used herein can be from, for example, fruit, animal origin, bacteria or fungi. The protease may have endo-activity and/or exo-activity or any combination thereof. It will be understood that suitable proteases for use in the process of the invention are available from commercial suppliers, such as Novozymes, Genencor, AB-Enzymes and DSM Food Specialities Amano, albeit without limitation thereto. Exemplary proteases are those of bacterial or fungal origin, such as from *Bacillus licheniformes* or *Aspergillus oryzae.*

The skilled person would readily understand that the term "acid", as used herein, refers to various water-soluble compounds with a pH of less than 7 that can be reacted with an alkali to form a salt. Examples of acids can be monoprotic or polyprotic and can comprise one, two, three, or more acid functional groups. Examples of acids include, but are not limited to, mineral acids, Lewis acids, acidic metal salts, organic acids, solid acids, inorganic acids, or any combination thereof. Preferably, acid treatment includes contacting the polysaccharide-based source material with a food grade acid, such as lactic acid, phosphoric acid, citric acid, malic acid, ascorbic acid, formic acid, fumaric acid, succinic acid, tartaric acid, gluconic acid and any combination thereof. Preferably, the acid, such as the food grade acid, has a concentration of about 0.1 to 5 M, and more preferably of about 0.5 to about 2 M.

In one particular embodiment, the food grade acid is or comprises gluconic acid, such as that derived at least in part from glucono delta-lactone. In this regard, it will be appreciated that glucono delta-lactone typically hydrolyses in aqueous solutions to produce gluconic acid.

Referring to the protein hydrolysis step, acid treatment is suitably carried out at a pH of about 2.0 to about 6.0, preferably about 3.0 to 4.0 or any range therein. In particular embodiments, acid treatment is carried out at a pH of about 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0 and any range therein. In certain preferred embodiments, acid treatment is carried out at a pH of about 4.2 to 4.4.

As would be readily understood by the skilled artisan, "alkali", as used herein, refers to various water-soluble compounds with a pH of greater than 7 that can be reacted with an acid to form a salt. By way of example, an alkali can include, but is not limited to, sodium hydroxide, potassium hydroxide, ammonium hydroxide, magnesium hydroxide and alkali metal salts such as, but not limited to, sodium carbonate and potassium carbonate.

In particular embodiments, the polysaccharide-based source material may be treated with one or more acids and optionally one or more alkalis in respect of the protein hydrolysis step. For example, the polysaccharide-based source material may be treated with 1, 2, 3, 4, 5, or more acids and/or alkalis.

For the protein hydrolysis step, the acid and optional alkali may be present in in an amount from about 0.1% to 15% or any range therein such as, but not limited to, about 0.3% to about 13%, or about 1% to about 10% by weight of the polysaccharide-based source material. In particular embodiments of the present invention, an acid and optional an alkali is present in the protein hydrolysis step in an amount of about 0.1%, 0.2%, 0.3%, 0.4%, 0.5%, 0.6%, 0.7%, 0.8%, 0.9%, 1%,

1.1%, 1.2%, 1.3%, 1.4%, 1.5%, 1.75%, 2%, 2.25%, 2.5%, 2.75%, 3%, 3.25%, 3.5%, 3.75%, 4%, 4.25%, 4.5%, 4.75%, 5%, 5.25%, 5.5%, 5.75%, 6%, 6.25%, 6.5%, 6.75%, 7%, 7.25%, 7.5%, 7.75%, 8%, 8.25%, 8.5%, 8.75%, 9%, 9.25%, 9.5%, 9.75%, 10%, 10.25%, 10.5%, 10.75%, 11%, 11.25%, 11.5%, 11.75%, 12%, 12.25%, 12.5%, 12.75%, 13%, 13.25%, 13.5%, 13.75%, 14%, 14.25%, 14.5%, 14.75%, 15% or any range therein, by weight of the polysaccharide-based source material. In certain embodiments of the present invention, an acid and optional alkali is present in the protein hydrolysis step in an amount of about 1% to about 2% by weight of the polysaccharide-based source material.

With respect to the protein hydrolysis step, heat treatment is suitably carried out at a temperature from about 40°C to 99°C, preferably about 55°C to about 90°C or any range therein, such as, but not limited to, about 65°C to about 85°C or about 45°C to about 80°C. In particular embodiments, heat treatment is carried out at a temperature of about 40°C, 41°C, 42°C, 43°C, 44°C, 45°C, 46°C, 47°C, 48°C, 49°C, 50°C, 51°C, 52°C, 53°C, 54°C, 55°C, 56°C, 57°C, 58°C, 59°C, 60°C, 61°C, 62°C, 63°C, 64°C, 65°C, 66°C, 67°C, 68°C, 69°C, 70°C, 71°C, 72°C, 73°C, 74°C, 75°C, 76°C, 77°C, 78°C, 79°C, 80°C, 81°C, 82°C, 83°C, 84°C, 85°C, 86°C, 87°C, 88°C, 89°C, 90°C, 91°C, 92°C, 93°C, 94°C, 95°C, 96°C, 97°C, 98°C, 99°C and any range therein. In certain preferred embodiments, heat treatment is carried out at a temperature of about 70°C to about 80°C.

In relation to the aforementioned aspects, the protein hydrolysis step is suitably carried out for a period of time from about 15 minutes to about 48 hours, preferably about 20 minutes to about 12 hours and more preferably from about 30 minutes to about 2 hours and any range therein. In particular embodiments, the protein hydrolysis step is carried out for a period of time of about 15 min, 20 min, 30 min, 40 min, 50 min, 1 hr, 1.25 hr, 1.5 hr, 1.75 hr, 2 hr, 3 hr, 4 hr, 5 hr, 6 hr, 7 hr, 8 hr, 9 hr, 10 hr, 11 hr, 12 hr, 13 hr, 14 hr, 15 hr, 16 hr, 17 hr, 18 hr, 19 hr, 20 hr, 21 hr, 22 hr, 23 hr, 24 hr, 25 hr, 26 hr, 27 hr, 28 hr, 29 hr, 30 hr, 31 hr, 32 hr, 33 hr, 34 hr, 35 hr, 36 hr, 37 hr, 38 hr, 39 hr, 40 hr, 41 hr, 42 hr, 43 hr, 44 hr, 45 hr, 46 hr, 47 hr, 48 hr and any range therein.

As generally used herein, the term "protein extraction" refers to the separation, removal and/or isolation of protein and more particularly hydrolysed protein, at least in part, from the polysaccharide-based source material, which may be performed by any method or means known in the art. Exemplary methods of protein extraction include gravity separation, centrifugation, size exclusion chromatography, hydrophobic interaction chromatography, ion exchange chromatography, free flow electrophoresis, metal binding, immunoaffinity chromatography and immunoprecipitation.

In some embodiments, the protein extraction step produces a second protein level that is at least about 50%, 40%, 30%, 20%, 15%, 10%, or 5% lower than that of the initial protein level of the polysaccharide-based starting material. In particular embodiments, the protein extraction step produces a second protein level that is at least about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50% and any range therein lower than said initial protein level.

With respect to the above, the degree of protein hydrolysis can be determined by any manner known to those skilled in the art (see, e.g., Petersen et al., Determination of the Degree of Hydrolysis (DH) based on OPA Reaction, ED-9512723 Novo Nordisk A/S, Dec. 1995; Frister et al., OPA method modified by use of N,N-dimethyl-2-mercaptoethylammonium chloride as thiol component, Fresenius J. Anal. Chem. 330 (1988) 631).

In a further aspect, the invention provides a polysaccharide-based ingredient prepared by the method of the aforementioned aspect.

With respect to the aforementioned aspects, the polysaccharide-based ingredient is preferably capable of or is adapted to modulate and/or control the water binding ability of a thickening agent, such as those hereinafter described. To this end, the polysaccharide-based ingredient is preferably able to produce specific degrees of viscosity inhibition of a liquid composition, such as those provided herein, comprising the polysaccharide-based ingredient and a thickening agent. Additionally, the polysaccharide-based ingredient may further control the rate and extent that their viscosity inhibition is released and/or reversed upon dilution of the liquid composition.

Accordingly, in another aspect, the invention provides a stable liquid of the third aspect.

The term *"thickening agent"* as used herein refers to those compounds provided herein that are used to increase the viscosity of a liquid mixture and/or solution, and in particular, those for use in food applications, including edible gums, vegetable gums and food-grade polysaccharides. Non-limiting examples of thickening agents include agar, alginic acid, carrageenan, guar gum, gum tragacanth, gum ghatti, microcrystalline cellulose, sodium carboxymethylcellulose, methyl cellulose, hydroxypropylmethylcellulose, hydroxyproylcellulose, methylethylcellulose, gum karaya, xanthan gum, locust bean gum, tara gum, psyllium seed gum, quince seed gum, a pectin, furcellaran, gellan gum, konjac, sodium alginate and any combination thereof.

Liquid compositions for thickening or increasing the viscosity of a foodstuff are known in the art. By way of example, US2004/0197456 (hereinafter "Holahan") describes a liquid thickener intended for people with swallowing disorders. The invention disclosed in Holahan, however, describes a liquid composition having a thickening agent concentrated to several times its intended usage level. Unlike the controlled-release technology described herein, the liquid thickener of Holahan comprises a thickening agent that already has its viscosity fully expressed therein and, so, which is fully hydrated even before addition to a foodstuff, after which Holahan’s liquid thickener is then simply added at a

volume such that the now diluted liquid thickener expresses the desired viscosity in the foodstuff.

In particular embodiments, the composition has a water activity of greater than 95%. It would be readily understood, that water activity or $a_w$ is defined as the ratio of the partial vapor pressure of water in a material to the standard state partial vapor pressure of water at the same temperature. Additionally, water generally migrates from areas of high water activity to areas of low water activity. For example, the liquid composition provided herein has a water activity in excess of 95% (e.g., about or in excess of 95.5%, 96%, 96.5%, 97%, 97.5%, 98%, 98.5%, 99%, 99.5% and any range therein), which then typically requires protection from atmospheres or environments with relative humidities of less than 95% so as to prevent the liquid composition from drying out during storage and before delivery or dispensing, such as by a pump dispenser or another sealed delivery system as are known in the art.

The liquid composition of the above aspects may be stored and/or delivered by any means known in the art. In particular embodiments, the liquid composition is stored and/or delivered by a container and pump dispenser arrangement, as are known in the art (see, e.g., PCT/AU2017/050966). In alternative embodiments, the liquid composition is stored and/or delivered by a sachet or the like, such as that provided herein.

Suitably, the liquid composition described herein when added in a desirable amount to an aqueous liquid or aqueous liquid solid mixture foodstuff does not alter particular desirable attributes thereof, such as the original flavour and/or colour of the foodstuff, that may be attractive to the consumer. In this regard, the liquid composition preferably makes little or no flavour and/or colour contribution to said foodstuff when added in a desirable amount thereto. Additionally, it is preferable that the amount of the liquid composition to be added to a foodstuff to achieve a desirable viscosity thereof is as small as possible so as to avoid diluting the flavour and/or colour characteristics of the foodstuff.

With regard to the present invention, the liquid composition described herein is suitably flowable. To this end, the liquid composition of the present invention suitably has a viscosity of less than 4000 cP and more preferably between about 2000 cP to about 4000 cP. Advantageously, a liquid composition of such a viscosity that may be dispensed easily, such as from a pump dispenser or a sachet, as well as being able to be dispersed with little or no agitation (i.e., a low shear mixing force) when added in a desired amount to an aqueous liquid or aqueous liquid solid mixture foodstuff. Further, the liquid composition of the invention is preferably concentrated and can accommodate a relatively higher percentage of thickening agent without losing the flowable character of the composition. This further enables easy and accurate dispensing of the liquid composition into the foodstuff of choice.

In certain embodiments of the aforementioned aspects, the liquid composition has a viscosity of about 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000, 2100, 2200, 2300, 2400, 2500, 2600, 2700, 2800, 2900, 3000, 3100, 3200, 3300, 3400, 3500, 3600, 3700, 3800, 3900, 4000 cP, or any range therein. Preferably, the liquid composition has a viscosity of between about 500 cP to about 1500 cP. More preferably, the liquid composition has a viscosity of between about 750cP to about 1250 cP.

The viscosity according to the methods of the invention is measured by a rheometer. The viscosity of the liquid composition may be measured by any means known in the art. By way of example, viscosity may be measured using a Bostwick Consistometer, a Brookfield Viscometer, or similar device. Preferably, viscosity is measured in absolute centipoise as provided by a rheometer, rather than relative centipoise as measured by a viscometer. It would be appreciated by the skilled artisan that a rheometer measurement represents the best and therefore standard method for determining foodstuff viscosity.

Suitably, the liquid composition described herein increases the viscosity of the aqueous liquid or aqueous liquid solid mixture foodstuff to greater than 95 cP. It is an advantage of the present approach that the inhibition of the expression, by the thickening agent, of its viscosity due to the polysaccharide-based ingredient is effectively lifted by gentle mixing of the liquid composition into the liquid or liquid solid foodstuff. This allows the thickening agent to quickly express its viscosity, due to the controlled release of the viscosity inhibitory effect of the polysaccharide-based ingredient on the thickening agent, and therefore aids in its easy and rapid incorporation into the foodstuff. This is an advantage over thickening agents which are substantially fully hydrated prior to being added to a foodstuff, such as that described in Holahan, and can therefore be challenging to incorporate into the foodstuff in a smooth and time efficient manner. Furthermore, the complete expression of viscosity by fully hydrated thickening agents is in itself an obstacle to the easy and rapid development of increased viscosity when it is diluted with a liquid or liquid solid foodstuff.

Therefore, it will be clear that in any of the aforementioned aspects, the thickening agent in the composition is preferably not fully hydrated prior to its addition to the foodstuff.

In certain embodiments, the viscosity of said foodstuff, upon addition of the liquid composition, is increased to at least 95, 100, 110, 120, 130, 140, 150, 175, 200, 250, 300, 350,400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850, 1900, 1950, 2000, 2050, 2100, 2150, 2200, 2250, 2300, 2350, 2400, 2450, 2500, 2550, 2600, 2650, 2700, 2750, 2800, 2850, 2900, 2950, 3000 cP, or any range therein.

For the purposes of the present invention, the thickening agent, may be present in an amount from about 3% to about 30% or any range therein such as, but not limited to, about 5% to about 15%, or about 7% to about 12% by weight of

the liquid composition. **In** particular embodiments of the present invention, the thickening agent is present in an amount of about 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0% or any range therein, by weight of the liquid composition. In certain embodiments of the present invention, the thickening agent is present in an amount of about 3% to about 20% by weight of the liquid composition.

For the present invention, the polysaccharide-based ingredient is suitably present in a high enough concentration that does not significantly contribute to the viscosity of the liquid composition. To this end, the polysaccharide-based ingredient described herein may be present in an amount from about 3% to about 30% or any range therein such as, but not limited to, about 5% to about 20%, or about 7.5% to about 17.5% by weight of the liquid composition.

In particular embodiments of the present invention, the polysaccharide-based ingredient described herein is present in an amount of about 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0%, 30.5%, 31.0%, 31.5%, 32.0%, 32.5%, 33.0%, 33.5%, 34.0%, 34.5%, 35.0%, 35.5%, 36.0%, 36.5%, 37.0%, 37.5%, 38.0%, 38.5%, 39.0%, 39.5%, 40.0% or any range therein, by weight of the liquid composition. In certain embodiments of the present invention, the polysaccharide-based ingredient described herein is present in an amount of about 3% to about 20% by weight of the liquid composition. If the concentration of the polysaccharide-based ingredient is below this range, the liquid composition typically forms a viscous solution and loses fluidity when the thickening agent is added.

Preferably, the polysaccharide-based ingredient is included in an amount such that the stable liquid composition has a lower viscosity than that of the liquid composition were it to comprise the thickening agent only with water or another suitable aqueous solution. More preferably, the polysaccharide-based ingredient decreases the viscosity of the stable liquid composition to at least a third of that of the liquid composition were it to comprise the thickening agent only with water or another suitable aqueous solution. In particular embodiments, the polysaccharide-based ingredient decreases the viscosity of the stable liquid composition to at least about 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60% or any range therein, of that of the liquid composition were it to comprise the thickening agent only with water or another suitable aqueous solution.

Suitably, the composition referred to herein is stable for at least six months and up to at least two years at room temperature. In this regard, the inventors have shown that the present liquid composition including the polysaccharide-based ingredient demonstrates little or no separation between its component materials (e.g., the polysaccharide-based ingredient and the thickening agent) after storage at room temperature for 6 months or more. This is in contrast to those liquid thickening agents known in the art. By way of example, US Patent 6,455,090 (hereinafter "Uzahashi") describes methods for producing a liquid thickening formulation, which can thicken when added to a liquid and is initially inhibited from forming viscous solutions or gels. The inventors claim that the invention can be added suitably to a liquid or semi-liquid foodstuff for a patient who has mastication and deglutition difficulties.

Nonetheless, the invention disclosed in Uzahashi is limited in that the thickening agent described therein exhibits neither microbial nor physically stability, but rather rapidly separates to create layers. Additionally, the thickening agent of Uzahashi fails to consistently and uniformly thicken liquid foods when added thereto. As such, the liquid thickener of Uzahashi has no practical utility in the management of swallowing disorders (dysphagia) so as to prevent or limit common co-morbidities of the condition. This lack of utility is two-fold. Firstly, the lack of physical stability and resultant separation of the solvent and gelling agents prohibits accurate dosing of Uzahashi's liquid thickener. As such the invention as disclosed cannot consistently guarantee to meet the required levels with regard to predetermined viscosity of the resultant thickened food. Secondly, patients such as those described herein are typically vulnerable populations. Indeed, the liquid thickener composition of Uzahashi is not microbiologically stable and thus should not be administered clinically to the intended population as described. Conversely, the liquid composition including the polysaccharide-based ingredient described herein successfully overcomes this limitation of the prior art by not separating to create layers and thus consistently imparts an accurate pre-determined viscosity to an aqueous liquid or aqueous liquid solid mixture foodstuff when added thereto (see, e.g., Table 3).

Because the composition of the present invention is stable, without significant degradation in the performance of the thickening agent, the viscosity remains constant for a commercially reasonable period of time. Accordingly, the formulation can be provided as a packaged product *per se*, such as in a metered pump dispenser or in a sachet, to the end user. To this end, the end user can reliably calculate the amount of the liquid composition of the invention to add to a food or beverage to achieve a desired end viscosity thereof. The liquid composition of the invention is then easily dispensed and

easily mixed into the foodstuff to give the desired end product.

As described earlier, the ability to package and use the liquid composition in this way is a result of the combined presence of the thickening agent and polysaccharide-based ingredient which inhibits the expression of the viscosity of the thickening agent until released through the application of low shear mixing and provides distinct benefits in use over traditional sachets of powdered or gel-like thickener which are notoriously difficult to measure out accurately, when the exact pack size is not appropriate, and to incorporate into liquid foodstuffs.

Stability of the liquid composition of the invention over time may be indicated by the retention of colour (if any), flavour (if any), separation (if any), microbiological spoilage (if any), viscosity and/or clarity of the liquid composition. Additionally or alternatively, stability of the liquid composition may be determined by the ability of the composition to impart viscosity consistently and repeatably to a predetermined level when added to a foodstuff. The stability of the liquid composition can be determined by using any of the techniques available to a person skilled in art of food science, including microbiological testing to measure the extent and rate of microbiological spoilage; visual inspection for physical changes such as separation and/or sedimentation; sensory evaluation to determine colour, flavour and/or clarity changes; and viscosity measurement using a Bostwick Consistometer, Brookfield Viscometer, a rheometer or similar device.

With respect to stability, the liquid composition of the invention may further comprise a food-grade preservative, as are well known in the art. Suitable food grade preservatives include, but are not limited to, gellan gum, vitamin E, potassium sorbate, sodium benzoate, sodiummetabisulphite, methyl paraben, EDTA, sulphur dioxide, nisin and propionic acid. In one preferred embodiment, the food-grade preservative is or comprises gellan gum. The amount of preservative in the liquid composition may range from about 0.001 to about 0.1 percent by weight of the total weight of the liquid composition.

Again, in regards to stability, the liquid composition described herein suitably is of a pH between about 3.0 and about 7.5 (e.g., 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5 and any range therein). Preferably, the pH of the liquid composition is between about 4 and 4.4. To this end, the acidic pH of the liquid composition may be achieved by any means known in the art, such as those hereinbefore described.

Suitably, the liquid composition described herein is added to an aqueous liquid or aqueous liquid solid mixture foodstuff for feeding to a subject suffering from a mastication and/or deglutition disease, disorder or condition. Preferably, the mastication and/or deglutition disease, disorder or condition is or comprises dysphagia. As such, it is preferable for this use that the liquid composition is separated into appropriate individual portions, such as sachets, or be pump dispensable.

It would be readily understood that dysphagia is a condition where the process of swallowing is impaired. During eating, this can lead to the entry of liquid or solid food into the trachea and subsequently the lungs of the sufferer potentially leading to aspiration pneumonia. Dysphagia can occur at any age, but is most common in the elderly, especially if they have suffered a stroke or have dementia. One management strategy for sufferers of dysphagia is to consume foods that are texture modified (i.e., thickened foods and beverages) that slow the swallowing reflex and allow the windpipe time to close before the food passes, thereby preventing aspiration of food.

Suitably, the composition is configured to produce substantially no change in an impedance level of the foodstuff when added thereto. As such, when added to a foodstuff it results in a medium of a known electrical impedance, which may be suitable for application in a diagnostic and/or prognostic setting, such as high resolution impedance manometry (HRIM). Accordingly, in particular embodiments, the foodstuff is or comprises a medium for use in determining a diagnosis and/or a prognosis of a subject suffering from a mastication and/or deglutition disease, disorder or condition, such as dysphagia.

Dysphagia symptoms are typically investigated by requiring a patient to swallow a contrast in front of an x-ray machine and imaging the swallow (video-fluoroscopy) so as to visualise the passage of contrast through the pharynx and oesophagus. This procedure is limited as it only provides a 'snap shot' of how a patient swallows. Furthermore, x-ray assessment is qualitative and video-fluoroscopy cannot assess the strength of a contraction or relaxation of muscles in the pharynx and oesophagus as well as how these may relate to the movement of swallowed content. The contractile state of the muscle can, however, be measured using a technique known as manometry. More recently, there has been an important advancement which enables measurement of pressure and the resulting flow of contents (impedance) in "high resolution" (i.e., HRIM). Utilising catheters that incorporate many closely-spaced pressure sensors combined with impedance electrodes, the contractile pressures and resulting flow can be seamlessly 'mapped' in space and time and flow maps constructed offering a biomechanically based means of swallow assessment for patients with dysphagia. However, this diagnostic capability requires a specialized bolus medium thickened to a consistent repeatable pre-determined level allowing accurate measurement and analysis of both pressure (manometry) and flow (impedance), but importantly does not appreciable effect the impedance level of the diagnostic bolus medium.

The diagnostic media for use in impedance studies, such as HRIM, generally include an electrolyte solution. It will be appreciated that the electrical impedance of such a diagnostic medium can be largely determined by the fixed charge density and therefore the concentration of charged particles therein. A liquid composition with practical utility in thickening such a diagnostic medium must generally be configured so as to maintain the impedance level thereof within a known

impedance range (e.g., 150-200 Ohm), over a range of viscosities or thicknesses (e.g., 150-900 cP) known to have clinical efficacy.

Without being bound by any theory, it is believed that the removal of a proteinaceous fraction from the polysaccharide-based ingredient eliminates, reduces or controls the concentration of charged proteins of the liquid composition. Thus, when added to a diagnositic medium, such as an aqueous electrolyte solution, the impedance of the resultant diagnostic medium demonstrates little or no change in impedance, as there is little or no increase in the level of charged particles therein.

In yet a further aspect, the invention provides a method for increasing the viscosity of an aqueous liquid or aqueous liquid solid mixture foodstuff, as described in the fourth aspect.

Suitably, the method further comprises the step of applying low-shear mixing to the foodstuff and the composition so as to promote increasing the viscosity of said foodstuff by the composition.

As generally used herein, the term "low shear mixing" refers to non-turbulent or minimally turbulent mixing, such as gentle mixing or stirring with a spoon or the like. It would be understood that low-shear mixing may be defined in terms of shear rates and typically is a function of a number of variables, such as mixing vessel configuration and mixing device speed.

It would be appreciated that the low-shear mixing is suitably of a value that is sufficient to promote the physical removal of the polysaccharide-based ingredient from its inhibitory interaction site on the one or plurality of thickening agents, so as to allow said thickening agents to exert their desired effect of increasing the relevant liquid or semi-liquid's foodstuff's viscosity. Accordingly, in particular embodiments the low-shear mixing comprises stirring at a speed of from about 10 rpm to about 40 rpm (e.g., about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 rpm or any range therein).

Suitably, the low-shear mixing is applied for about 60 seconds or less to achieve a maximal or near-maximal increase in viscosity of the foodstuff. Preferably, the low-shear mixing is applied for about 10 to about 40 seconds (e.g., about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 seconds or any range therein) to achieve a maximal or near maximal viscosity of the foodstuff.

In certain embodiments, the viscosity of the foodstuff is suitably increased to greater than 95 cP.

In referring to the above aspect, the foodstuff of increased viscosity is suitably for feeding a subject suffering from a mastication and/or deglutition disease, disorder or condition. Preferably, the mastication and/or deglutition disease, disorder or condition is or comprises dysphagia.

In yet another aspect, the invention provides a method of producing a stable liquid composition, as described in the fifth aspect.

Suitably, the stable liquid composition is that hereinbefore described.

Manufacture of the stable liquid composition of the invention can include the step of heating the polysaccharide-based ingredient and/or the one or plurality of thickening agents when present, for example, in a suitable liquid carrier, such as an aqueous carrier. The heated composition can then be hot-fill packaged, or cooled prior to packaging.

The present method may include the step of preparing an aqueous solution or suspension of the polysaccharide-based ingredient. In this regard, the aqueous solution may have a dry mass content of the polysaccharide-based ingredient from about 0.1 to about 60 wt%, based on the total amount of the aqueous solution of suspension.

Similarly, the present method may include the step of preparing an aqueous solution or suspension of the thickening agent. In this regard, the aqueous solution may have a dry mass content of the thickening agent from about 0.1 to about 60 wt%, based on the total amount of the aqueous solution of suspension.

The method of the current aspect may optionally include the step of adding one or more excipients or additives to the stable liquid composition, such as colours, flavours, protein (animal and plant), dietary fibres, vitamins and minerals, humectants, for example glycerol and sorbitol, fats and oils, emulsifiers, acidity regulators, antioxidants, low calorie bulking agents, firming agents, flavour enhancers, foaming agents, gelling agents, preservatives, sequestrants and stabilisers.

Throughout the specification the aim has been to describe the preferred embodiments of the invention without limiting the invention to any one embodiment or specific collection of features. It will therefore be appreciated by those of skill in the art that, in light of the instant disclosure, various modifications and changes can be made in the particular embodiments exemplified without departing from the scope of the present invention.

Any reference to publications cited in this specification is not an admission that the disclosures constitute common general knowledge in Australia.

In order that the invention may be more readily understood and put into practice, one or more preferred embodiments thereof will now be described, by way of example only.

**Example 1: Method of Manufacturing a Polysaccharide-based ingredient**

The objective of the present example was to analyse an embodiment of a polysaccharide-based ingredient of the

invention with respect to protein content and make-up as well as the proteinaceous fraction removed therefrom.

### Methods

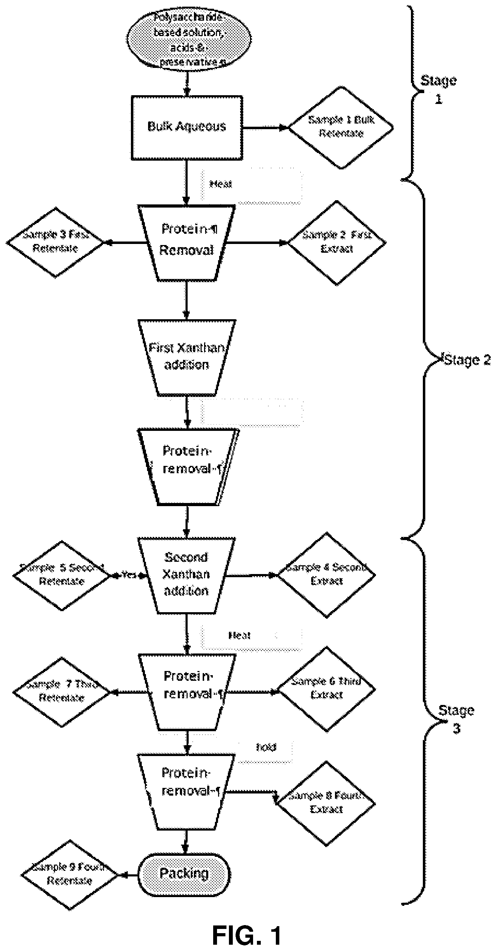

Eight samples from various stages in the manufacturing process of a food thickening composition as detailed in Figure 1 were analysed. Aliquots (15-20 g) from each sample were placed into 8 containers, respectively, and shipped to Massey University's Nutrition laboratory for total nitrogen quantification determined by the Dumas method (updated version of the Kjeldahl method) (Leco, AOAC 968.06).

### Results

### Mass balance

A mass balance of inputs and outputs entering and leaving the system during the process described in Figure 1 is summarised in Table 1. A total of 43.9kg was removed as a result of acid heat hydrolysis.

**Table 1** Mass balance of inputs and outputs entering and leaving the system during manufacturing process of a food thickening composition

| | KG | | | |
|---|---|---|---|---|
| **STAGE** | Input | Output 1 (Extract) | Output 2 | Accumulation (Retentate) |
| **1** | 9843.30 | 8.70 | | 9834.60 |
| **2** | 1327.60 | 15.20 | 224.00 | 10923.00 |
| **3** | 527.50 | 20.00 | 168.00 | 11262.50 |

### Total nitrogen analysis

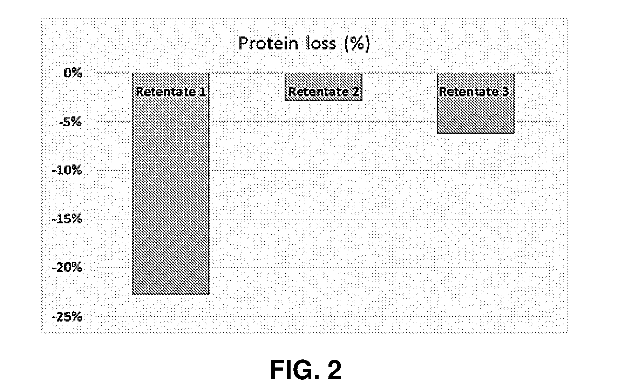

The protein content of the retentate samples was approximated by multiplying the total nitrogen obtained analytically (Tables 2) with the Jones conversion factor, 6.25. (Jones, 1931). The percentage reduction in protein content of the retentate at each stage is illustrated in Figure 2. The protein contents across the samples ranged from 0.0031 g/g to 0.0063 g/g where sample 4 (2nd collection extract) contained the highest protein content and sample 5 (2nd collection retentate) contained the lowest protein content. The majority of the protein was extracted from the polysaccharide based ingredient (and/or an intermediate thereof) after the initial 2 hour step of acid and heat hydrolysis. The removal of significant amounts of protein underpins the technology facilitating the subsequent application of the food thickening composition. Specifically: stable site specific inhibition of a hydrated viscosity inhibited xanthan solution; and altered electrical impedance facilitating diagnostic application as described.

**Table 2** Protein content of retentate samples was approximated by multiplying the total nitrogen obtained analytically

| *Sampling point from Figure 1* | *Sample* | *CI name* | *N %* | *Protein % (g/100g)* |
|---|---|---|---|---|
| 1 | Bulk retentate | CI-TSC-8 | 0.06 | 0.38 |
| 3 | First retentate | CI-TSC-3 | 0.05 | 0.31 |
| 5 | Second retentate | CI-TSC-5 | 0.07 | 0.44 |
| 7 | Third retentate | CI-TSC-6 | 0.08 | 0.50 |
| 9 | Fourth retentate | CI-TSC-7 | 0.08 | 0.50 |

**References**

AOAC 968.06-1969, Protein (Crude) in animal feed. Dumas method. Jones, D. B. (1931). Factors for converting percentages of nitrogen in foods and fees into percentages of proteins. Circular No. 183. US Department of Agriculture, Washington, DC.

**Example 2: Assessment of Impedance of a Diagnostic Medium Thickened by the Invention**

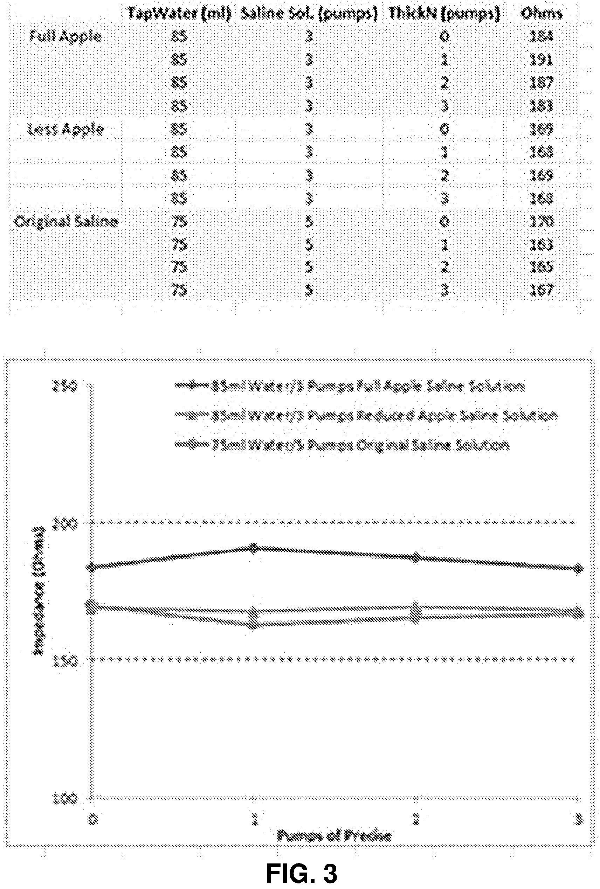

The objective of the present Example was to assess the effect the addition of an embodiment of a liquid composition having a polysaccharide-based ingredient of the invention has on the electrical impedance of a diagnostic medium. As can be observed in Figure 3, the present study compared 10 mL of the diagnostic bolus medium across 4 consistencies (thickened, thickened to level 150, level 400 and level 900) thickened with various concentrations of a liquid composition produced using the polysaccharide-based ingredient. Parameters that are influenced by the level of impedance (e.g. UES Opening; Bolus Presence Time) are very stable across the different consistencies.

**Table 3** Target ranges for the viscosity of liquids thickened by the disclosed invention in milliPascal seconds (mPa.s) for the three consistency levels as per Speech Pathology Profession Guidelines

| Classification | Level | Description | Target viscosity range* | Level of precision achieved with current invention |
|---|---|---|---|---|
| Mildly thick | 150 | nectar consistency | 110-190 mPa.s | 130-170 mPa.s |
| Moderately thick | 400 | honey consistency | 300-500 mPa.s | 350-450 mPa.s |
| Extremely think | 900 | pudding consistency | 750 -1000 mPa.s | 825-975 mPa.s |

**Example 3: Protein analysis data of protein extracted from the polysaccharide based ingredient**

In this Example, samples taken during the process of producing the ingredient, as well as the initial hydrocolloid and the final product, were analysed in regards to their protein/peptide content and profile using SDS-PAGE and LC-MS analysis of bands recovered from the gel. The results obtained for the individual samples were compared against the starting material to determine if any changes occurred during processing.

**Materials and Methods**

**SDS-PAGE protocol**

Eight samples from various steps in the manufacturing process of a food thickening product described in Example 1 were taken. Aliquots (approximately 200 mg) of each sample were weighed into Eppendorf tubes, and diluted with water to give a final weight of 1000 mg. Based on the previously undertaken total protein determination, this should result in protein concentrations between 0.6 and 1 mg/mL of sample (see table 1). Prior to the SDS PAGE, 5 μL sample were mixed with 5 μL LDS sample buffer, 2 μL β-mercaptoethanol and 8 μL water to give a final volume of 20 μL. These solutions were heated for 10 minutes at 70 °C. After allowing these samples to cool down, 15 μL of each sample were applied to the wells of a pre-cast NuPAGE gel (NuPAGE, Bis-Tris, 4-12%, 1.0 mm). The electrophoresis was carried out over 35 minutes at room temperature (starting voltage: 200 V, starting current: 90 mA). The power was supplied by a Pharmacia biotech electrophoresis power pack (EPS 600). The gel was stained with Coomassie blue. As a molecular weight standard, the SeeBlue Plus2 pre-stained protein ladder (Invitrogen) was added.

**LC/MS analysis of peptides in gel bands and solutions**

*Sample preparation*

Gel bands were diced and destained with acetonitrile: 50mM ammonium bicarbonate (1:1), then dehydrated with acetonitrile, and submerged in 10mM dithiothreitol. A 5ul aliquot of the two solution samples was diluted with 45ul of 50mM ammonium bicarbonate and DTT added to 10mM final concentration. All samples were heated at 56 °C for 15 minutes. The

gel band supernatant was then replaced with 50mM iodoacetamide, while iodoacetamide was added to the solution samples to 50mM final concentration. All samples were incubated in the dark at room temperature for 30 minutes. Gel pieces were dehydrated with acetonitrile, dried and re-swelled with 12.5 ng/μL sequencing-grade modified porcine trypsin (Promega), while 1ug of sequencing-grade modified porcine trypsin was added to the solution samples. All samples were digested in a chilled microwave (CEM Discover) at 45 °C using 15W of power for 60 minutes. Digests were acidified with 1 μL of 50% formic acid.

The two solution digests were desalted and cleaned up on 10mg Oasis HLB SPE cartridges, eluting with 300ul of 50% acetonitrile. Extracts were dried in a vacuum centrifuge to ~20ul. Solution extracts were diluted 20-fold, and gel band digests 3-fold in 0.1% formic acid for LC-MS/MS analysis.

*LC-MS/MS analysis*

A 2ul aliquot of each diluted sample was injected onto a 0.3x 10mm trap column packed with 3um Reprosil C18 media (Dr Maisch) for desalting before being separated on a 0.075 x 150 mm picofrit column (New Objective) packed in-house with 3um Reprosil C18 media using a 30 minute gradient of 0.1% formic acid in water and 0.1% formic acid in acetonitrile at 300nl/min.

The picofrit spray was directed into a TripleTOF 6600 Quadrupole-Time-of-Flight mass spectrometer (Sciex, Framingham, MA, USA) scanning from 350-1600 m/z for 200ms, followed by 50ms MS/MS scans on the 35 most abundant multiply-charged peptides (m/z 100-1600) with a dynamic exclusion time of 12 seconds. The mass spectrometer and HPLC system were under the control of the Analyst TF 1.7 software package (Sciex). The resulting data from each pool were searched against the aforementioned protein sequence database using ProteinPilot version 5.0 (Sciex) with the following parameters: Sample Type, Identification; Search Effort, Thorough; Cys Alkylation, Iodoacetamide; Digestion, Trypsin; ID Focus, Biological modifications and Amino Acid Substitutions (allows for up to two amino acids per peptide sequence to be substituted). Manual de novo sequencing was performed for seven high intensity MS/MS spectra that were not matched above. Extracted Ion Chromatograms for these peptides (+/-0.015Da) were created using PeakView 2.2 (AB Sciex).

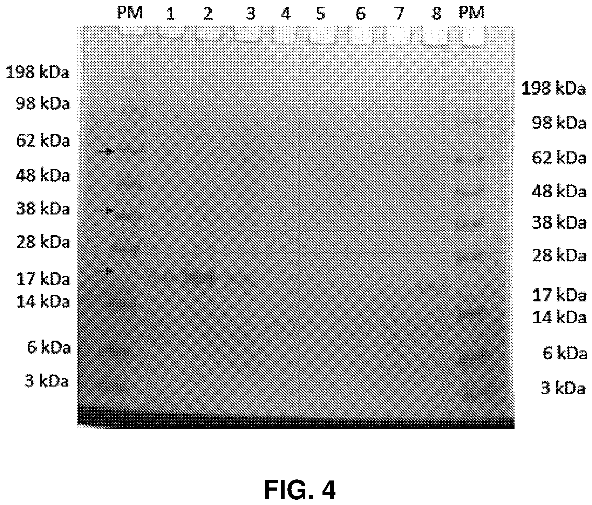

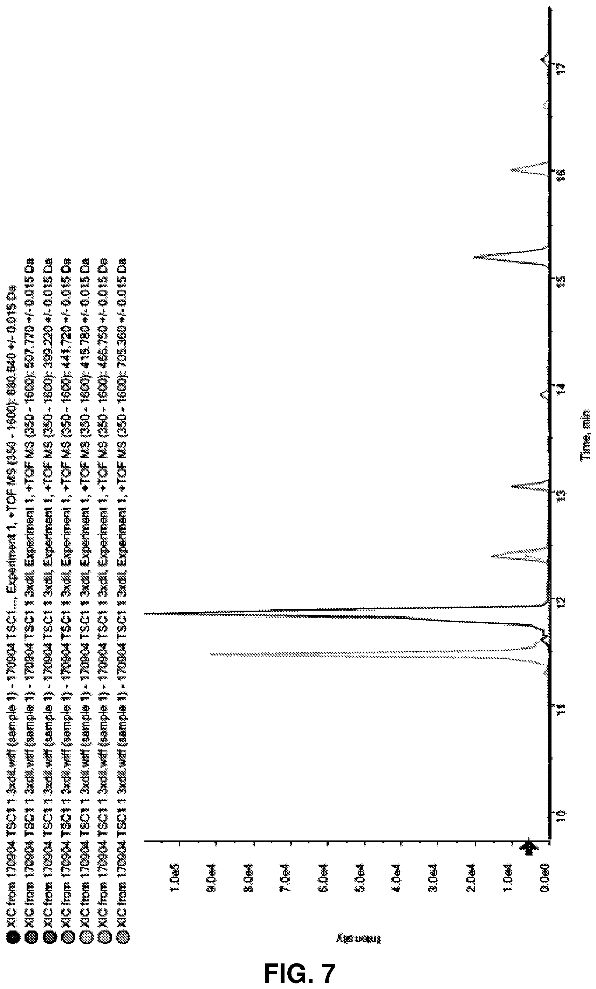

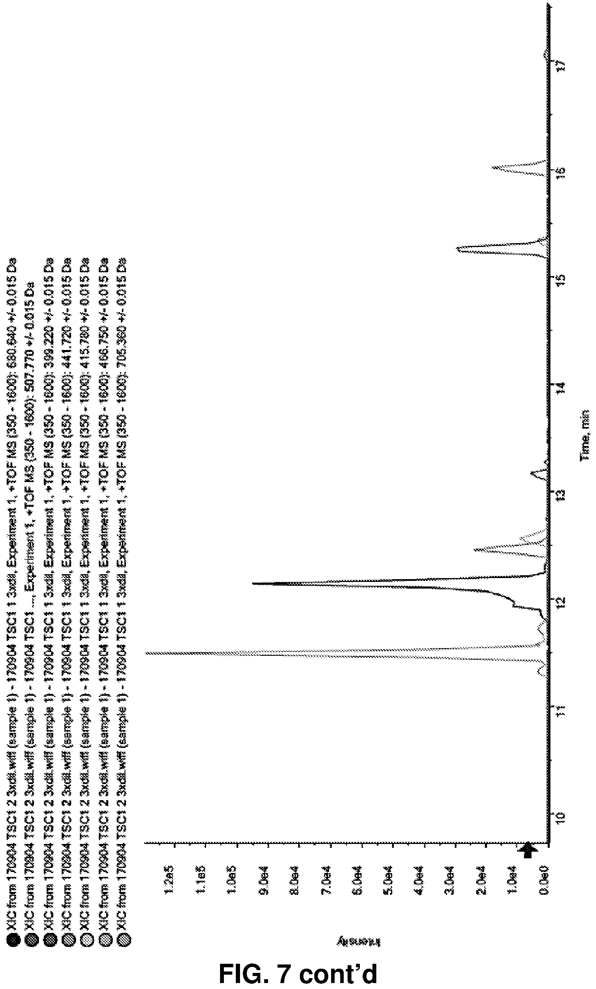

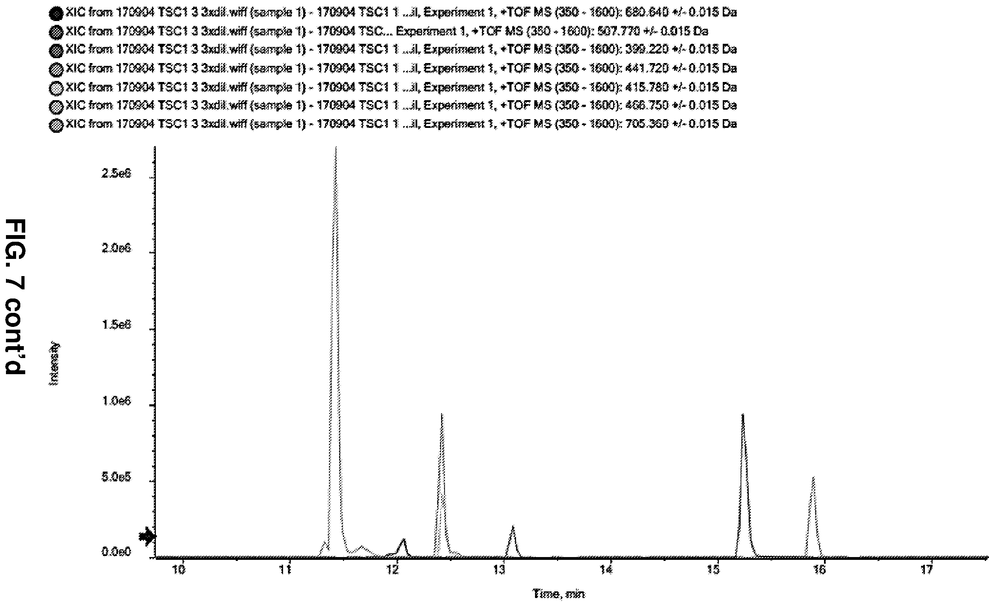

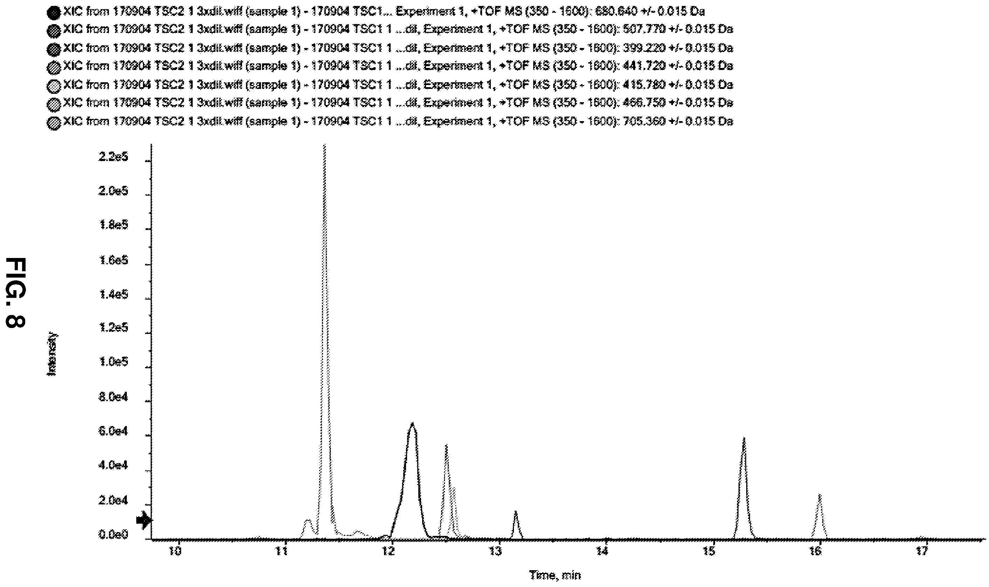

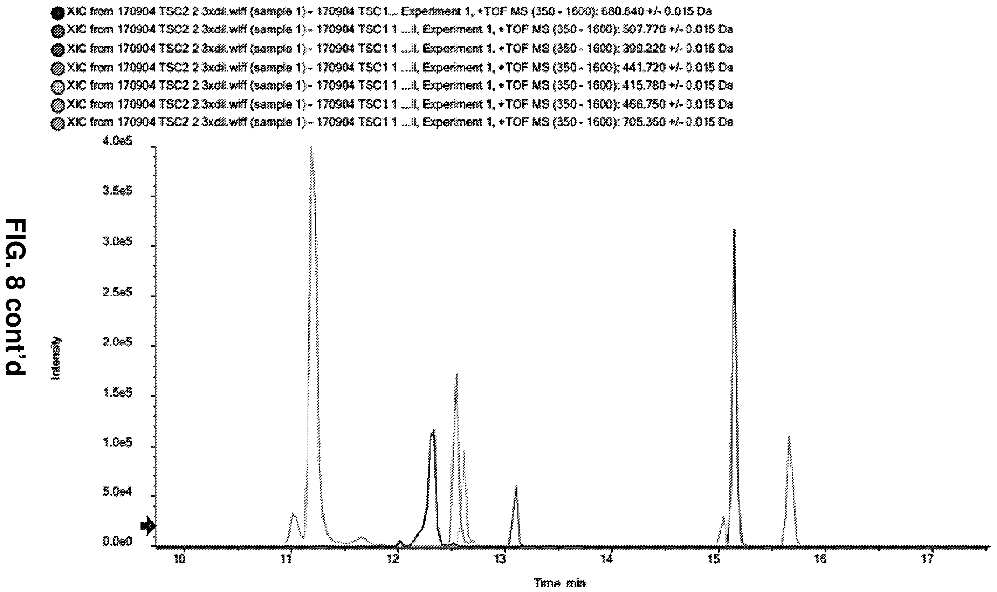

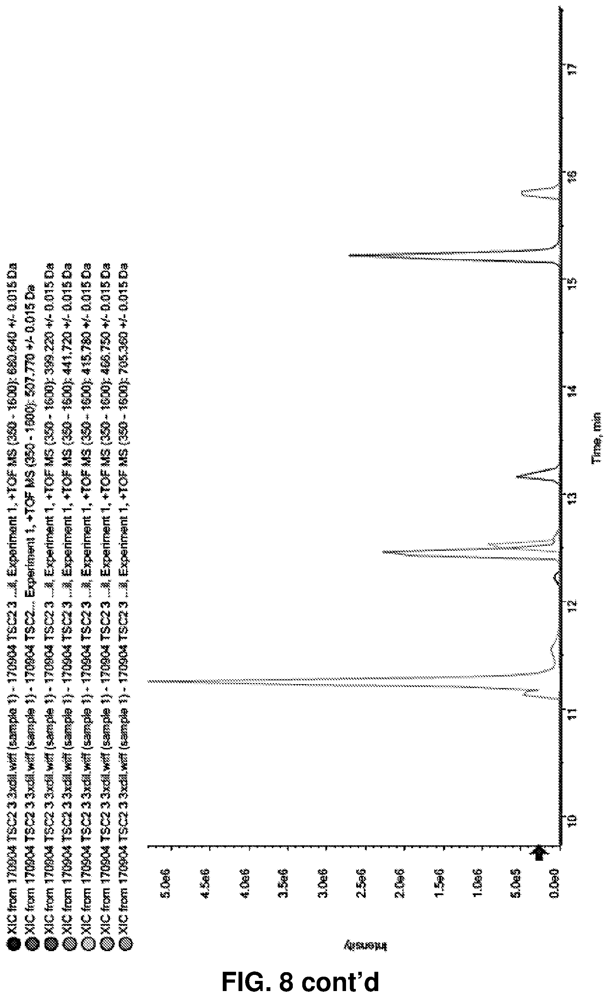

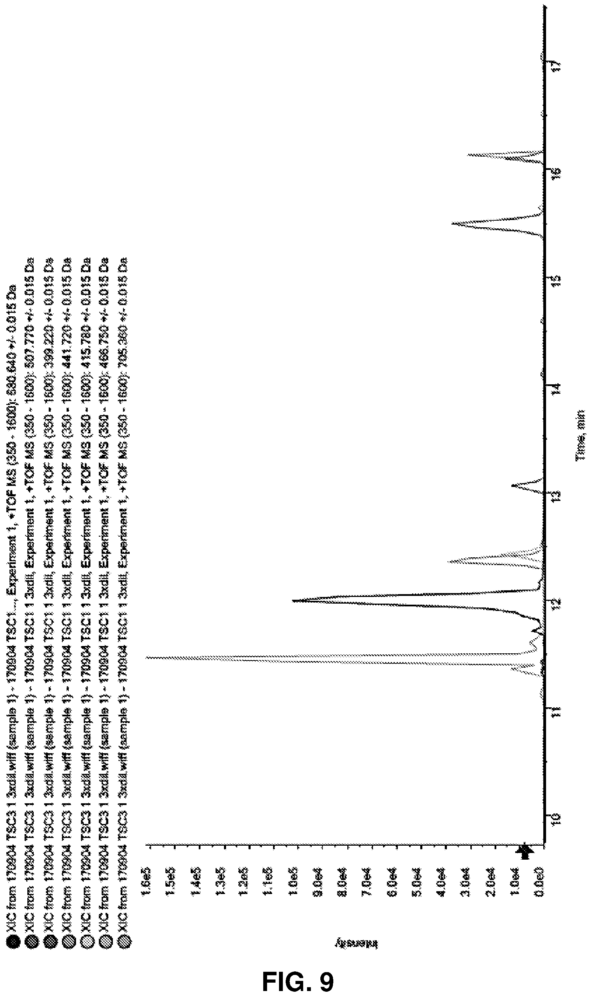

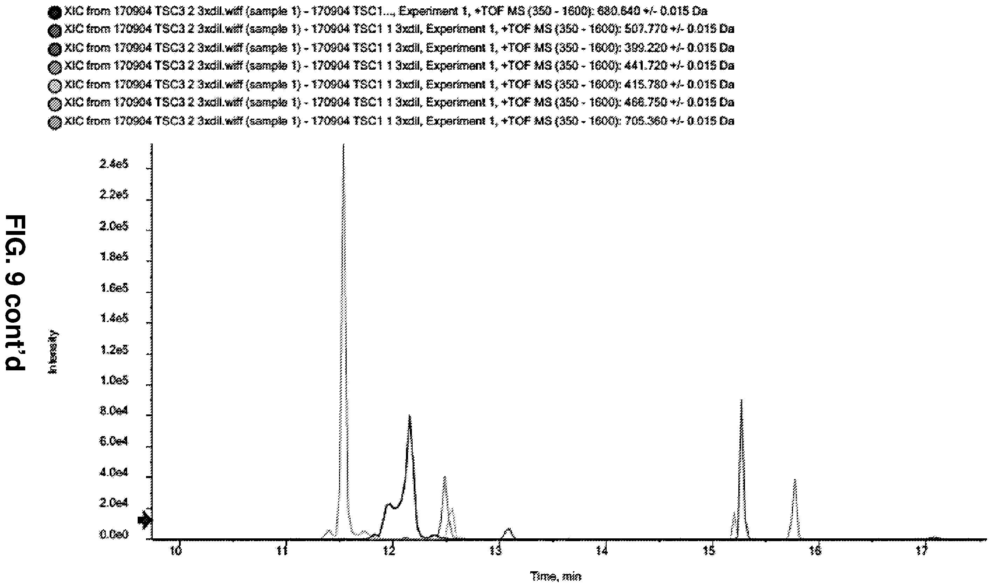

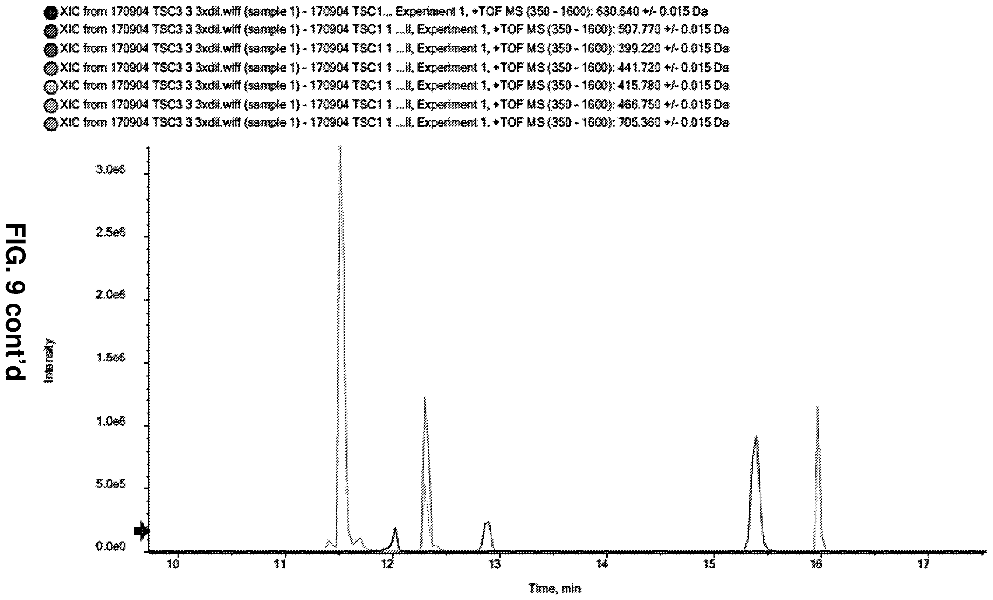

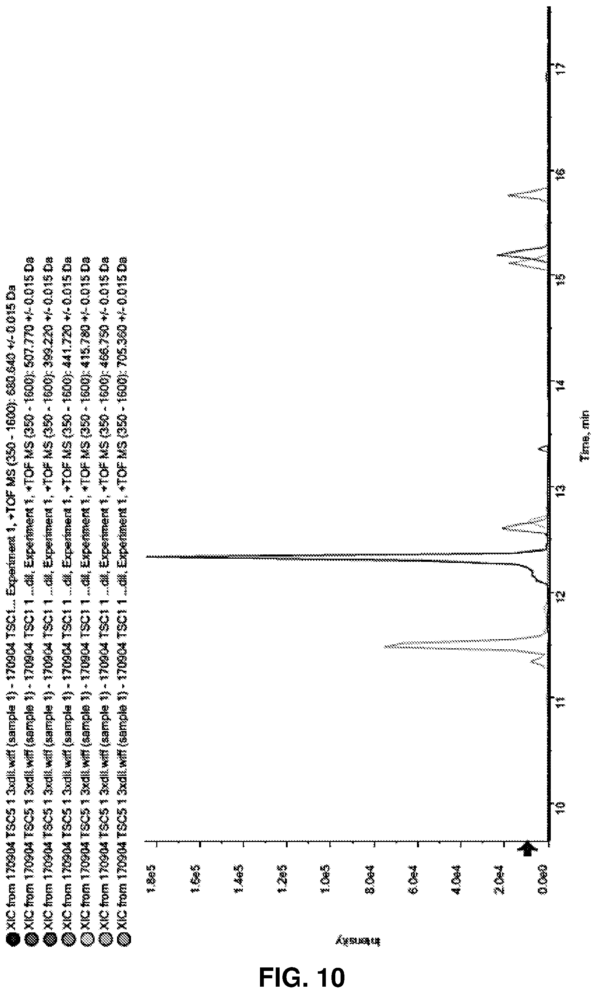

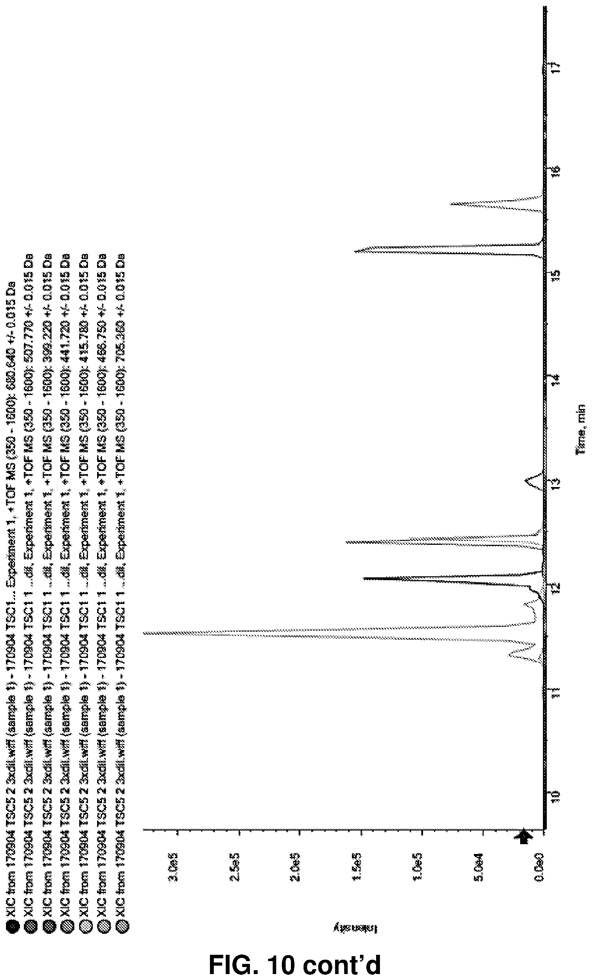

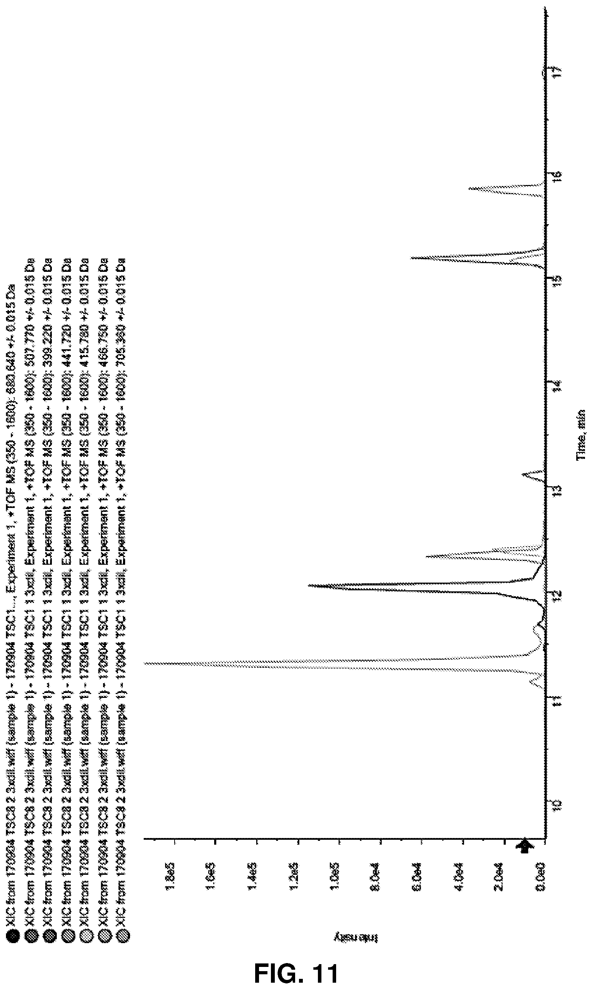

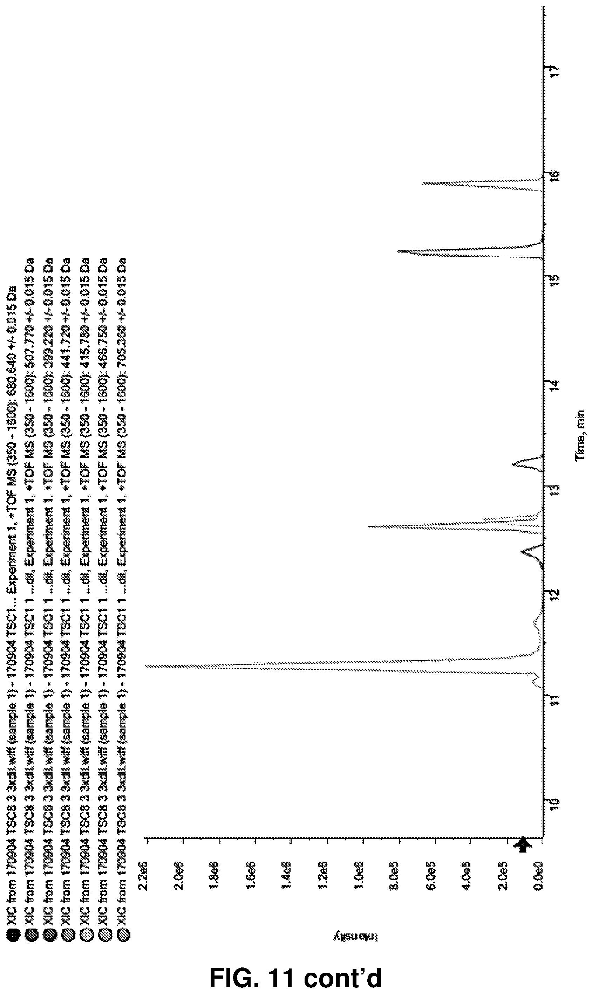

Figures 7 to 11 summarise the extracted ion chromatograms generated for the bands extracted from SDS PAGE according to Figure 4.

**Determination of protein/peptide profile in hydrocolloids**

Determination of protein/peptide profile using SDS-PAGE

Eight samples from various steps of the process were studied (see Table 4). Initially, the total protein content of the samples was approximated by determining the total nitrogen content in the sample1 and multiplying this value with the Jones conversion factor (JF= 6.25)2. The results summarised in Table 4, show that the samples contain between 3.1 and 6.3 mg protein/g sample.

The protein content was calculated on the basis of the total nitrogen content determined according to the Dumas method at the Massey University Nutrition Laboratory.

**Table 4: Protein content in the hydrocolloid samples**

| Sample number* | Sample description | CI sample number | Protein in mg/g |
|---|---|---|---|
| 1 | Bulk retentate 1 (initial hydrocolloid) | TSC 8 | 3.80 |
| 2 | Bulk retentate 2 | TSC 3 | 3.10 |
| 3 | First extract | TSC 1 | 3.80 |
| 4 | Bulk retentate 3 | TSC 5 | 4.40 |
| 5 | Second extract | TSC 2 | 6.30 |
| 6 | Bulk retentate 4 | TSC 6 | 5.00 |
| 7 | Third extract | TSC 4 | 5.60 |
| 8 | Bulk retentate 5 (commercial product) | TSC 7 | 5.00 |
| *According to process sequence outlined in Figure 1 | | | |

During the process, a slight increase in the total protein content was observed with the extracts having a higher

content when compared with the retentates taken from the same process stage (e.g. bulk retentate 2 and first extract, see Table 4).

In this regard, we highlight the reduction in protein from the initial untreated hydrocolloid to the first extract and prior to the addition of gum (e.g., xanthan gum) to the treatment process. We further note that the protein levels of the third and fourth retentates (i.e., samples 6 and 8) rise owing to the addition of xanthan gum and hence additional protein to the treatment process.

For the SDS PAGE analysis, solutions of approximately 200 mg/g in water were prepared of each sample. Based on the previously undertaken total protein determination, this should result in protein concentrations between 0.6 and 1 mg/mL of sample. Samples CI-TSC 4, 6 and 7 did not give clear solutions, but formed solid heterogenous gels making the pipetting and further processing of the samples challenging. The samples were treated and subjected to SDS PAGE according to Laemmli. The resulting gel is shown in Figure 4.

In the starting material (lane 8, TSC 8), the first two extracts (lane 1, TSC1 and 2, TSC 2) and bulk retentate 2 one strong band at 20 kDa and two faint bands at 40 and 60 kDa are visible. In contrast, only very faint staining was observed for the bulk retentate 3 (lane 5, TSC 5) and no protein was detected in the third extract (lane 4, TSC 4), the bulk retentate 4 (lane 6, TSC 6) and the commercial product (lane 7, TSC 7). In this regard, we highlight the loss of the 60 kDa protein in the third retentate following the initial heat treatment and protein extraction steps. It is hypothesised that the removal of this larger molecular weight protein fraction positively impacts stability of the polysaccharide-based ingredient of the invention.

According to the earlier results, these latter samples contain between 0.44 and 0.56 % protein (see Table 4) and should have given enough protein to be detectable on the gel under the electrophoresis conditions used. However, as mentioned above, these samples formed very viscous gels when mixed with water. This made the processing of the samples and the transfer to the gel challenging, and could have resulted in not enough protein being loaded onto the gel.

LC/MS analysis of proteins/peptides

LC/MS analysis of the proteins/peptides was undertaken by the Centre for Genomics and Proteomics at the School of Biological Sciences at the University of Auckland, New Zealand. The generated data for each pool were searched against the protein sequence database the using ProteinPilot (see Materials and Methods section above).

For the analysis, the bands visible on the SDS PAGE gel after staining (see red arrows in Fig. 4) were carefully cut out using a surgical knife and placed individually in labelled Eppendorf vials. Overall, thirteen samples were taken as outlined in Table 5.

The samples were processed and analysed according to the standard protocol as outlined in detail above. The resulting peptides were compared with a protein sequence database with the entries for sequences from the species potentially present in the samples as well as entries for possible contaminants (e.g. human keratins).

**Table 5: gel bands extracted from SDS PAGE gel for LC/MS analysis**

| Sample number | Sample description | CI sample number | Band number | Molecular weight (kDa) | Band ID |
|---|---|---|---|---|---|
| 1 | Bulk retentate 1 | TSC 8 | 1<br>2<br>3 | 60<br>40<br>20 | TSC8-1<br>TSC8-2<br>TSC8-3 |
| 2 | Bulk retentate 2 | TSC 3 | 1<br>2<br>3 | 60<br>40<br>20 | TSC3-1<br>TSC3-2<br>TSC3-3 |
| 3 | First extract | TSC 1 | 1<br>2<br>3 | 60<br>40<br>20 | TSC1-1<br>TSC1-2<br>T3C1-3 |
| 4 | Bulk retentate 3 | TSC 5 | 1<br>2 | 40<br>20 | TSCS-1<br>TSC5-2 |
| 5 | Second extract | TSC 2 | 1<br>2<br>3 | 60<br>40<br>20 | TSC2-1<br>TSC2-2<br>TSC2-3 |

The database search generated many matches to human keratins, porcine trypsin (which was used for processing the samples) and some plant derived proteins (Table 6). However, it was observed that the more intense peptides were not automatically identified (see Table 6). Even a wider search of databases including other plants

(containing 3.5 million entries) did not generate a match.

**Table 6: List of proteins identified by ProteinPilot for gel band sample TSC2-3.**

| Protein Name (or best BLAST match name) | Species | Unused Score* | Peptides Conf >95% |
|---|---|---|---|
| Keratin 1 | Homo sapiens | 71.6 | 45 |
| Keratin 10 | Homo sapiens | 32.0 | 19 |
| Beta Trypsin | Sus scrofa | 23.9 | 37 |
| Keratin, type II cytoskeletal 6C | Homo sapiens | 23.1 | 17 |
| Peroxidase | Soy Bean | 11.8 | 9 |
| Protein P21 | Soy Bean | 7.8 | 4 |
| Actin-like | Soy Bean | 6.3 | 3 |
| Acidic endochitinase-like | Soy Bean | 2.0 | 2 |
| Leucine-rich repeat extensin-like | Soy Bean | 2.0 | 1 |
| 14 kDa proline-rich protein | Soy Bean | 2.0 | 1 |
| Beta-galactosidase | Escherichia coli | 2.0 | 1 |
| *unused score is a measure of unique peptide evidence for each protein. B-galactosidase is used to calibrate the mass spectrometer. | | | |

Manual sequencing of the seven more intense peptides generated the proposed peptide sequences summarised in Table 7. These were used to search the databases for all plant species. While some partial matches were generated, there was no clear indication of a particular plant protein as being the source of all of these peptides suggesting a protein sequence far different from anything currently publicly available.

In addition to this, the above described treatment process resulted in a polysaccharide-based ingredient demonstrated novel protein fractions. Furthermore, we hypothesise that removal of the original 60 kDa protein fraction in the final retentate (and from the third retentate onwards) results in an improvement in product stability, and particularly in respect of separation.

**Table 7: Proposed manually derived *de* novo sequences for the abundant unmatched peptides.**

| m/z | z | RT (min) | Proposed de novo sequence | ppm error |
|---|---|---|---|---|
| 680.6341 | 3 | 12.1 | (AS or SA)SGANTPSGPYTHD....* | n/a |
| 507.7728 | 2 | 15.3 | NPEWLVTR | 1.6 |
| 399.2250 | 3 | 13.2 | No clear result | n/a |
| 441.7236 | 2 | 12.4 | LVFCSEK | 1.1 |
| 415.7782 | 2 | 12.5 | VKPLVFK | -0.2 |
| 466.7511 | 2 | 11.2 | LLVTDDEK | 1.5 |
| 705.3631 | 2 | 15.9 | NGGNYYLVSVPAR | 1.2 |
| *Not completely sequenced due to ambiguity I the low mass part of fragment ion spectrum | | | | |

**Table 8: List of proteins identified by ProteinPilot for gel band sample TSC8 OR.**

| Protein Name (or best BLAST match name) | Species | Unused Score* | Peptides Conf >95% |
|---|---|---|---|
| Peroxidase | Soy Bean | 22.7 | 14 |
| Peroxidase | Soy Bean | 21.2 | 17 |
| Protein P21-like | Soy Bean | 17.1 | 14 |
| Beta Trypsin | Sus scrofa | 15.8 | 34 |
| Beta-xylosidase/alpha-L-arabinafuranosidase 1 | Soy Bean | 6.6 | 5 |
| Galactose oxidase-like | Soy Bean | 6.1 | 7 |
| Peroxidase | Soy Bea n | 5.5 | 5 |
| Leucine-rich repeat family protein / extensin | Soy Bean | 4.3 | 4 |

(continued)

| Protein Name (or best BLAST match name) | Species | Unused Score* | Peptides Conf >95% |
|---|---|---|---|
| Beta-xylosidase/alphe-L-arabinofuranesidase 2 | Soy Bean | 4.0 | 5 |
| Keratin 1 | Homo sapiens | 4.0 | 2 |
| Glucan endo-1,3-beta-glucosidase-like protein | Soy Bean | 4.0 | 2 |
| Elongation factor Tu | Sphingomonas | 3.5 | 2 |
| Uncharacterized protein | Soy Bean | 3.2 | 1 |
| Cysteine proteinase inhibitor | Soy Bean | 2.9 | 3 |
| Reticuline oxidase-like protein | Soy Bean | 2.8 | 1 |
| Reticuline oxidase-like protein | Soy Bean | 2.3 | 2 |
| Uncharacterized protein | Soy Bean | 2.1 | 2 |
| Peroxidase | Soy Bean | 2.1 | 2 |
| Aspartic protease | Soy Bean | 2.0 | 1 |
| Peroxidase | Soy Bean | 2.0 | 2 |
| Acidic endothitinase-like | Soy Bean | 2.0 | 1 |
| Reticuline oxidase-like protein | Soy Bean | 2.0 | 1 |
| Peroxidase | Soy Bean | 2.0 | 14 |
| Leucine-rich repeat extensin-like protein 6 | Soy Bean | 2.0 | 2 |
| Formin-like protein | Soy Bean | 2.0 | 1 |
| 14 kDa proline-rich protein | Soy Bean | 2.0 | 1 |
| Basic endochitinase-like | Soy Bean | 2.0 | 2 |
| Putative lipid-transfer protein | Soy Bean | 2.0 | 1 |
| Gibberellin-regulated protein 13-like | Soy Bean | 2.0 | 1 |
| *unused score is a measure of unique peptide evidence for each protein. B-galactosidase is used to calibrate the mass spectrometer. | | | |

Overall, the relative abundances of the seven major peptides and the generally consistent appearance of these peptides across all samples has confirmed that the major (unidentified) protein present in these samples is to be found in all 13 preparations, but with a notable observed decrease in abundance of one peptide from the lower protein molecular weight gel bands being consistent with a loss of a particular region of the parent protein as might be expected (see ion chromatograms in Figures 7 to 11). As noted above, we hypothesise that the removal of this particular protein fraction imparts stability to the application.

**Conclusions**

The LC/MS analysis of the samples taken during the processing of generated a profile containing seven major peptides. Extensive search of known plant protein databases did not result in a match for these peptides.

**Example 4: Stability Comparison of Uzuhashi Embodiment Versus the Present Invention**

The present example relates to the second method described in the Detailed Description of the Preferred Embodiments (column 4 line 26) of US Patent 6,455,090 (hereinafter "Uzuhashi") and compares it with the formulation of Example 1 above of an acidified and preservatised solution of thickening agents and viscosity inhibiting polysaccharide as provided for by the present invention.

Uzahashi describes methods for producing a liquid thickening agent, which can thicken when added to a liquid and is initially inhibited from forming viscous solutions or gels. The inventors claim that the invention can be added suitably to a liquid or semi-liquid foodstuff for a patient who has mastication and deglutition difficulties.

*Microbiological Stability*

**Table 1. Time (in weeks) to Develop Evidence of Microbiological Growth***

| Uzuhashi Embodiment | Example 1 Embodiment |
|---|---|
| Less than 1 (about 2days at 25C) | Greater than 52 at 25C |

*The development of microbiological growth is first detected by the presence of microbial fermentation as demonstrated by the appearance of gas (e.g., $CO_2$) being produced and the development of "off" odours in the solution.

**Physical Stability**

Physical stability of the respective formulations was evidenced by separation of the thickening agent(s) from the viscosity inhibiting polysaccharide. To this end, the viscosity (as measured by Bostwick Consistometer after 30 seconds of flow) of a 20g sample of the liquid thickener was taken from the bottom of the container and mixed onto 100mls of water (Note: increasing Bostwick readings indicate a reduction (thinning) of viscosity).

**Table 2. Physical stability over time.**

| Weeks of Storage | Uzuhashi Embodiment | Example 1 Embodiment |
|---|---|---|
| 1 | 16.5cms | 16.0cm |
| 2 | 18cms | 16.0cm |
| 3 | 22cms | 16.0cm |
| 4 | >24cms* | 16.0cm |
| 5 | >24cms* | 16.0cm |
| 6 | >24cm* | 16.0cm |

*A Bostwick Consistometer's limit of reading for thin fluids is 24cms.

After 4 weeks, the Uzuhashi embodiment continued to produce thinner viscosities even though the Bostwick reading shows no change. After 8 weeks, the separation layer at the bottom of the Uzuhashi embodiment only contained a clear layer of the viscosity inhibiting polysaccharide and no thickening agent, whereas the formulation of Example 1 remains physically stable for greater than 52 weeks.

Accordingly, the invention disclosed in Uzahashi is limited in that the thickening agent described therein exhibits neither microbial nor physically stability. As such, the liquid thickener of Uzahashi has no practical utility in the management of swallowing disorders (dysphagia) so as to prevent or limit common co-morbidities of the condition. This lack of utility is two-fold. Firstly, the lack of physical stability and resultant separation of the solvent and gelling agents prohibits accurate dosing of Uzahashi's liquid thickener. **In** addition to the above, Uzahashi's liquid thickener further demonstrates a reduced ability to thicken liquid or liquid-solid foodstuffs consistently and uniformly when compared to the liquid thickener of Example 1.

Given the above, the invention as disclosed in Uzahashi cannot consistently guarantee to meet the required levels with regard to predetermined viscosity of the resultant thickened food. Secondly, patients such as those described herein are typically vulnerable populations. As such they are governed by legislative instruments such as the *NSW Food Authority - Guidelines for food service to vulnerable persons.* The liquid thickener composition of Uzahashi is not microbiologically stabile and thus could not be administered clinically to the intended population as described.

## Claims

**1.** A polysaccharide-based ingredient for use in preparing a food thickening composition comprising:

a polysaccharide-based source material selected from the group consisting of a *Larix occidentalis* polysaccharide extract, a *Larix laricina* polysaccharide extract, an Acacia tree polysaccharide extract, a *Larix decidua* polysaccharide extract, a *Larix sibirica* polysaccharide extract and any combination thereof;
wherein the polysaccharide-based source material has been subjected to a protein hydrolysis step, wherein the hydrolysis step includes:

(a) a heat treatment step carried out at a temperature of from about 55°C to about 90° C; and
(b) an acid treatment step carried out at a pH of about 3 to about 5.

2. The polysaccharide-based ingredient of Claim 1, wherein the acid treatment step includes contacting the polysaccharide-based source material with a food grade acid, wherein optionally the food grade acid is selected from the group consisting of lactic acid, phosphoric acid, citric acid, malic acid, ascorbic acid, formic acid, fumaric acid, succinic acid, tartaric acid, gluconic acid and any combination thereof.

3. The polysaccharide-based ingredient of Claim 1 or Claim 2, wherein the heat treatment step has been carried out at a temperature of from about 70°C to about 80°C.

4. The polysaccharide-based ingredient of any one of the preceding claims, wherein the acid treatment step has been carried out at a pH of about 3.9 to 4.4.

5. The polysaccharide-based ingredient of any one of the preceding claims, wherein the protein hydrolysis step has been carried out for a period of time from: (a) about 15 minutes to about 30 hours; or (b) about 30 minutes to about 2 hours.

6. The polysaccharide-based ingredient of any one of the preceding claims, wherein the polysaccharide-based ingredient has: (a) a protein content that is less than 2 wt% and is capable of modulating and/or controlling the water binding ability of a thickening agent; or (b) a protein content that is less than 1 wt% and is capable of modulating and/or controlling the water binding ability of a thickening agent.

7. A method of preparing a polysaccharide-based ingredient for use in preparing a food thickening composition including the steps of:

(i) providing a polysaccharide-based source material selected from the group consisting of a *Larix occidentalis* polysaccharide extract, a *Larix laricina* polysaccharide extract, an Acacia tree polysaccharide extract, a *Larix decidua* polysaccharide extract, a *Larix sibirica* polysaccharide extract and any combination thereof; and
(ii) hydrolysing a portion of protein of the polysaccharide-based source material, wherein the hydrolysis step includes:

(a) a heat treatment step carried out at a temperature of from about 55°C to about 90° C; and
(b) an acid treatment step carried out at a pH of about 3 to about 5;

to thereby prepare the polysaccharide-based ingredient.

8. The method of Claim 7, wherein the heat treatment step is carried out at a temperature of from about 70°C to about 80°C.

9. The method of Claim 7 or Claim 8, wherein the acid treatment step is carried out at a pH of about 3.9 to 4.4.

10. The method of any one of Claims 7 to 9, wherein the protein hydrolysis step is carried out for a period of time from: (a) about 15 minutes to about 30 hours; or (b) about 30 minutes to about 2 hours.

11. A stable liquid composition having a viscosity of less than 4000 mPa s (4000 cP) when measured by a rheometer comprising:

(i) one or a plurality of thickening agents, wherein the thickening agent is selected from the group consisting of agar, alginic acid, carrageenan, guar gum, gum tragacanth, gum ghatti, microcrystalline cellulose, sodium carboxymethylcellulose, methyl cellulose, hydroxypropylmethylcellulose, hydroxypropylcellulose, methylethylcellulose, gum karaya, xanthan gum, locust bean gum, tara gum, psyllium seed gum, quince seed gum, a pectin, furcellaran, gellan gum, konjac, sodium alginate and any combination thereof and wherein the thickening agent is present in an amount from about 3% to about 30% by weight of the stable liquid composition; and
(ii) a polysaccharide-based ingredient comprising a polysaccharide-based source material selected from the group consisting of a *Larix occidentalis* polysaccharide extract, a *Larix laricina* polysaccharide extract, an Acacia tree polysaccharide extract, a *Larix decidua* polysaccharide extract, a *Larix sibirica* polysaccharide extract and any combination thereof, wherein the polysaccharide-based source material has been subjected to a protein hydrolysis step;

wherein addition of the composition to an aqueous liquid or aqueous liquid solid mixture foodstuff increases the viscosity of said foodstuff.

**12.** A method for increasing the viscosity of an aqueous liquid or aqueous liquid solid mixture foodstuff, the method including the steps of:

(a) adding to the foodstuff a stable liquid composition having a viscosity of less than 4000 mPa s (4000 cP) when measured by a rheometer comprising:

(i) one or a plurality of thickening agents, wherein the thickening agent is selected from the group consisting of agar, alginic acid, carrageenan, guar gum, gum tragacanth, gum ghatti, microcrystalline cellulose, sodium carboxymethylcellulose, methyl cellulose, hydroxypropylmethylcellulose, hydroxypropylcellulose, methylethylcellulose, gum karaya, xanthan gum, locust bean gum, tara gum, psyllium seed gum, quince seed gum, a pectin, furcellaran, gellan gum, konjac, sodium alginate and any combination thereof and wherein the thickening agent is present in an amount from about 3% to about 30% by weight of the stable liquid composition; and
(ii) a polysaccharide-based ingredient comprising a polysaccharide-based source material selected from the group consisting of a *Larix occidentalis* polysaccharide extract, a *Larix laricina* polysaccharide extract, an Acacia tree polysaccharide extract, a *Larix decidua* polysaccharide extract, a *Larix sibirica* polysaccharide extract and any combination thereof, wherein the polysaccharide-based source material has been subjected to a protein hydrolysis step; and

(b) mixing the foodstuff and the composition so as to promote increasing the viscosity of said foodstuff by the composition.

**13.** A method of producing a stable liquid composition, including the steps of:

(i) providing a polysaccharide-based ingredient comprising a polysaccharide-based source material selected from the group consisting of a *Larix occidentalis* polysaccharide extract, a *Larix laricina* polysaccharide extract, an Acacia tree polysaccharide extract, a *Larix decidua* polysaccharide extract, a *Larix sibirica* polysaccharide extract and any combination thereof, wherein the polysaccharide-based source material has been subjected to a protein hydrolysis step;
(ii) adding one or a plurality of thickening agents to the polysaccharide-based ingredient, wherein the thickening agent is selected from the group consisting of agar, alginic acid, carrageenan, guar gum, gum tragacanth, gum ghatti, microcrystalline cellulose, sodium carboxymethylcellulose, methyl cellulose, hydroxypropylmethylcellulose, hydroxypropylcellulose, methylethylcellulose, gum karaya, xanthan gum, locust bean gum, tara gum, psyllium seed gum, quince seed gum, a pectin, furcellaran, gellan gum, konjac, sodium alginate and any combination thereof and wherein the thickening agent is present in an amount from about 3% to about 30% by weight of the stable liquid composition; and
(iii) mixing the mixture of step (ii) to thereby produce the stable liquid composition.

**14.** The stable liquid composition of Claim 11, wherein the polysaccharide-based ingredient is that according to any one of Claims 1 to 6.

**15.** The method of Claim 12 or Claim 13, wherein the polysaccharide-based ingredient is that according to any one of Claims 1 to 6.

## Patentansprüche

**1.** Bestandteil auf Polysaccharidbasis zur Verwendung bei der Herstellung einer Lebensmittel-Eindickzusammensetzung, umfassend:

ein Ausgangsmaterial auf Polysaccharidbasis, ausgewählt aus der Gruppe bestehend aus einem *Larix-occidentalis-Polysaccharidextrakt,* einem *Larix-laricina*-Polysaccharidextrakt, einem Akazienbaum-Polysaccharidextrakt, einem *Larix-decidua*-Polysaccharidextrakt, einem *Larix-sibirica*-Polysaccharidextrakt und einer beliebigen Kombination davon;
wobei das Ausgangsmaterial auf Polysaccharidbasis einem Proteinhydrolyseschritt unterzogen worden ist,

wobei der Hydrolyseschritt Folgendes enthält:

(a) einen Wärmebehandlungsschritt, durchgeführt bei einer Temperatur von etwa 55 °C bis etwa 90 °C; und
(b) einen Säurebehandlungsschritt, durchgeführt bei einem pH-Wert von etwa 3 bis etwa 5.

**2.** Bestandteil auf Polysaccharidbasis nach Anspruch 1, wobei der Säurebehandlungsschritt Inkontaktbringen des Ausgangsmaterials auf Polysaccharidbasis mit einer Säure in Lebensmittelqualität enthält, wobei die Säure in Lebensmittelqualität optional aus der Gruppe ausgewählt ist, die aus Milchsäure, Phosphorsäure, Zitronensäure, Äpfelsäure, Ascorbinsäure, Ameisensäure, Fumarsäure, Bernsteinsäure, Weinsäure, Gluconsäure und einer beliebigen Kombination davon besteht.

**3.** Bestandteil auf Polysaccharidbasis nach Anspruch 1 oder Anspruch 2, wobei der Wärmebehandlungsschritt bei einer Temperatur von etwa 70 °C bis etwa 80 °C durchgeführt worden ist.

**4.** Bestandteil auf Polysaccharidbasis nach einem der vorhergehenden Ansprüche, wobei der Säurebehandlungsschritt bei einem pH-Wert von etwa 3,9 bis 4,4 durchgeführt worden ist.

**5.** Bestandteil auf Polysaccharidbasis nach einem der vorhergehenden Ansprüche, wobei der Proteinhydrolyseschritt für einen Zeitraum von: (a) etwa 15 Minuten bis etwa 30 Stunden; oder (b) etwa 30 Minuten bis etwa 2 Stunden durchgeführt worden ist.

**6.** Bestandteil auf Polysaccharidbasis nach einem der vorhergehenden Ansprüche, wobei der Bestandteil auf Polysaccharidbasis Folgendes aufweist: (a) einen Proteingehalt, der weniger als 2 Gew.-% beträgt und in der Lage ist, die Wasserbindungsfähigkeit eines Eindickmittels zu modulieren und/oder zu steuern; oder (b) einen Proteingehalt, der weniger als 1 Gew.-% beträgt und in der Lage ist, die Wasserbindungsfähigkeit eines Eindickmittels zu modulieren und/oder zu steuern.

**7.** Verfahren zur Herstellung eines Bestandteils auf Polysaccharidbasis zur Verwendung bei der Herstellung einer Lebensmittel-Eindickzusammensetzung, die folgenden Schritte enthaltend:

(i) Bereitstellen eines Ausgangsmaterials auf Polysaccharidbasis, ausgewählt aus der Gruppe bestehend aus einem *Larix-occidentalis-Polysaccharidextrakt,* einem *Larix-laricina*-Polysaccharidextrakt, einem Akazienbaum-Polysaccharidextrakt, einem *Larix-decidua*-Polysaccharidextrakt, einem *Larix-sibirica-Polysaccharidextrakt* und einer beliebigen Kombination davon; und
(ii) Hydrolysieren eines Teils des Proteins des Ausgangsmaterials auf Polysaccharidbasis, wobei der Hydrolyseschritt Folgendes enthält:

(a) einen Wärmebehandlungsschritt, durchgeführt bei einer Temperatur von etwa 55 °C bis etwa 90 °C; und
(b) einen Säurebehandlungsschritt, durchgeführt bei einem pH-Wert von etwa 3 bis etwa 5;

um dadurch den Bestandteil auf Polysaccharidbasis herzustellen.

**8.** Verfahren nach Anspruch 7, wobei der Wärmebehandlungsschritt bei einer Temperatur von etwa 70 °C bis etwa 80 °C durchgeführt wird.

**9.** Verfahren nach Anspruch 7 oder Anspruch 8, wobei der Säurebehandlungsschritt bei einem pH-Wert von etwa 3,9 bis 4,4 durchgeführt wird.

**10.** Verfahren nach einem der Ansprüche 7 bis 9, wobei der Proteinhydrolyseschritt für einen Zeitraum von: (a) etwa 15 Minuten bis etwa 30 Stunden; oder (b) etwa 30 Minuten bis etwa 2 Stunden durchgeführt wird.

**11.** Stabile flüssige Zusammensetzung mit einer Viskosität von weniger als 4000 mPa s (4000 cP), wenn gemessen durch ein Rheometer, umfassend:

(i) ein oder eine Vielzahl von Eindickmitteln, wobei das Eindickmittel ausgewählt ist aus der Gruppe bestehend aus Agar, Alginsäure, Carrageen, Guargummi, Traganthgummi, Ghatti-Gummi, mikrokristalliner Cellulose, Natriumcarboxymethylcellulose, Methylcellulose, Hydroxypropylmethylcellulose, Hydroxypropylcellulose, Methylethylcellulose, Karaya-Gummi, Xanthan-Gummi, Johannisbrotgummi, Tara-Gummi, Flohsamengummi,

Quittensamengummi, einem Pektin, Furcellaran, Gellangummi, Konjak, Natriumalginat und einer beliebigen Kombination davon und wobei das Eindickmittel in einer Menge von etwa 3 % bis etwa 30 % bezogen auf das Gewicht der stabilen flüssigen Zusammensetzung vorliegt; und
(ii) einen Bestandteil auf Polysaccharidbasis, umfassend ein Ausgangsmaterial auf Polysaccharidbasis, das ausgewählt ist aus der Gruppe bestehend aus einem *Larix-occidentalis*-Polysaccharidextrakt, einem *Larix-laricina*-Polysaccharidextrakt, einem Akazienbaum-Polysaccharidextrakt, einem *Larix-decidua*-Polysaccharidextrakt, einem *Larix-sibirica*-Polysaccharidextrakt und einer beliebigen Kombination davon, wobei das Ausgangsmaterial auf Polysaccharidbasis einem Proteinhydrolyseschritt unterzogen worden ist;

wobei die Hinzufügung der Zusammensetzung zu einem wässrigen flüssigen oder wässrigen flüssig-festen Mischlebensmittel die Viskosität des genannten Lebensmittels erhöht.

**12.** Verfahren zum Erhöhen der Viskosität eines wässrigen flüssigen oder wässrigen flüssig-festen Mischlebensmittels, wobei das Verfahren die folgenden Schritte enthält:

(a) Hinzufügen zu dem Lebensmittel einer stabilen flüssigen Zusammensetzung mit einer Viskosität von weniger als 4000 mPa s (4000 cP), wenn gemessen durch ein Rheometer, umfassend:

(i) ein oder eine Vielzahl von Eindickmitteln, wobei das Eindickmittel ausgewählt ist aus der Gruppe bestehend aus Agar, Alginsäure, Carrageen, Guargummi, Traganthgummi, Ghatti-Gummi, mikrokristalliner Cellulose, Natriumcarboxymethylcellulose, Methylcellulose, Hydroxypropylmethylcellulose, Hydroxypropylcellulose, Methylethylcellulose, Karaya-Gummi, Xanthan-Gummi, Johannisbrotgummi, Tara-Gummi, Flohsamengummi, Quittensamengummi, einem Pektin, Furcellaran, Gellangummi, Konjak, Natriumalginat und einer beliebigen Kombination davon und wobei das Eindickmittel in einer Menge von etwa 3 % bis etwa 30 % bezogen auf das Gewicht der stabilen flüssigen Zusammensetzung vorliegt; und
(ii) einen Bestandteil auf Polysaccharidbasis, umfassend ein Ausgangsmaterial auf Polysaccharidbasis, das ausgewählt ist aus der Gruppe bestehend aus einem *Larix-occidentalis*-Polysaccharidextrakt, einem *Larix-laricina*-Polysaccharidextrakt, einem Akazienbaum-Polysaccharidextrakt, einem *Larix-decidua*-Polysaccharidextrakt, einem *Larix-sibirica*-Polysaccharidextrakt und einer beliebigen Kombination davon, wobei das Ausgangsmaterial auf Polysaccharidbasis einem Proteinhydrolyseschritt unterzogen worden ist; und

(b) Mischen des Lebensmittels und der Zusammensetzung, um ein Erhöhen der Viskosität des Lebensmittels durch die Zusammensetzung zu fördern.

**13.** Verfahren zum Erzeugen einer stabilen flüssigen Zusammensetzung, die folgenden Schritte enthaltend:

(i) Bereitstellen eines Bestandteils auf Polysaccharidbasis, umfassend ein Ausgangsmaterial auf Polysaccharidbasis, das ausgewählt ist aus der Gruppe bestehend aus einem *Larix-occidentalis-Polysaccharidextrakt,* einem *Larix-laricina*-Polysaccharidextrakt, einem Akazienbaum-Polysaccharidextrakt, einem *Larix-decidua*-Polysaccharidextrakt, einem *Larix-sibirica*-Polysaccharidextrakt und einer beliebigen Kombination davon, wobei das Ausgangsmaterial auf Polysaccharidbasis einem Proteinhydrolyseschritt unterzogen worden ist;
(ii) Hinzufügen eines oder einer Vielzahl von Eindickmitteln zu dem Bestandteil auf Polysaccharidbasis, wobei das Eindickmittel ausgewählt ist aus der Gruppe bestehend aus Agar, Alginsäure, Carrageen, Guargummi, Traganthgummi, Ghatti-Gummi, mikrokristalliner Cellulose, Natriumcarboxymethylcellulose, Methylcellulose, Hydroxypropylmethylcellulose, Hydroxypropylcellulose, Methylethylcellulose, Karaya-Gummi, Xanthan-Gummi, Johannisbrotgummi, Tara-Gummi, Flohsamengummi, Quittensamengummi, einem Pektin, Furcellaran, Gellangummi, Konjak, Natriumalginat und einer beliebigen Kombination davon und wobei das Eindickmittel in einer Menge von etwa 3 % bis etwa 30 % bezogen auf das Gewicht der stabilen flüssigen Zusammensetzung vorliegt; und
(iii) Mischen der Mischung aus Schritt (ii), um dadurch die stabile flüssige Zusammensetzung zu erzeugen.

**14.** Stabile flüssige Zusammensetzung nach Anspruch 11, wobei der Bestandteil auf Polysaccharidbasis derjenige nach einem der Ansprüche 1 bis 6 ist.

**15.** Verfahren nach Anspruch 12 oder Anspruch 13, wobei der Bestandteil auf Polysaccharidbasis derjenige nach einem der Ansprüche 1 bis 6 ist.

## Revendications

**1.** Ingrédient à base de polysaccharide destiné à être utilisé dans la préparation d'une composition épaississante alimentaire comprenant :

un matériau source à base de polysaccharide choisi dans le groupe constitué d'un extrait polysaccharidique de *Larix Occidentalis,* d'un extrait polysaccharidique de *Larix laricina,* d'un extrait polysaccharidique d'acacia, d'un extrait polysaccharidique de *Larix decidua,* d'un extrait polysaccharidique de *Larix sibirica* et de toute combinaison de ceux-ci ;
dans lequel le matériau source à base de polysaccharide a été soumis à une étape d'hydrolyse de protéine, dans lequel l'étape d'hydrolyse comprend :

(a) une étape de traitement thermique réalisée à une température d'environ 55 °C à environ 90 °C ; et
(b) une étape de traitement acide réalisée à un pH d'environ 3 à environ 5.

**2.** Ingrédient à base de polysaccharide de la revendication 1, dans lequel l'étape de traitement acide comprend la mise en contact du matériau source à base de polysaccharide avec un acide de qualité alimentaire, dans lequel éventuellement l'acide de qualité alimentaire est choisi dans le groupe constitué de l'acide lactique, de l'acide phosphorique, de l'acide citrique, de l'acide malique, de l'acide ascorbique, de l'acide formique, de l'acide fumarique, de l'acide succinique, de l'acide tartrique, de l'acide gluconique et de toute combinaison de ceux-ci.

**3.** Ingrédient à base de polysaccharide de la revendication 1 ou la revendication 2, dans lequel l'étape de traitement thermique a été réalisée à une température d'environ 70 °C à environ 80 °C.

**4.** Ingrédient à base de polysaccharide de l'une quelconque des revendications précédentes, dans lequel l'étape de traitement acide a été réalisée à un pH d'environ 3,9 à 4,4.

**5.** Ingrédient à base de polysaccharide de l'une quelconque des revendications précédentes, dans lequel l'étape d'hydrolyse de protéine a été réalisée pendant une durée : (a) d'environ 15 minutes à environ 30 heures ; ou (b) d'environ 30 minutes à environ 2 heures.

**6.** Ingrédient à base de polysaccharide de l'une quelconque des revendications précédentes, dans lequel l'ingrédient à base de polysaccharide présente : (a) une teneur en protéine inférieure à 2 % en poids et est capable de moduler et/ou de contrôler la capacité de liaison à l'eau d'un agent épaississant ; ou (b) une teneur en protéine inférieure à 1 % en poids et est capable de moduler et/ou de contrôler la capacité de liaison à l'eau d'un agent épaississant.

**7.** Procédé de préparation d'un ingrédient à base de polysaccharide destiné à être utilisé dans la préparation d'une composition épaississante alimentaire comprenant les étapes de :

(i) fourniture d'un matériau source à base de polysaccharide choisi dans le groupe constitué d'un extrait polysaccharidique de *Larix occidentalis,* d'un extrait polysaccharidique de *Larix laricina,* d'un extrait polysaccharidique d'acacia, d'un extrait polysaccharidique de *Larix decidua,* d'un extrait polysaccharidique de *Larix sibirica* et de toute combinaison de ceux-ci ; et
(ii) hydrolyse d'une partie de protéine du matériau source à base de polysaccharide, dans lequel l'étape d'hydrolyse comprend :

(a) une étape de traitement thermique réalisée à une température d'environ 55 °C à environ 90 °C ; et
(b) une étape de traitement acide réalisée à un pH d'environ 3 à environ 5 ;

pour préparer ainsi l'ingrédient à base de polysaccharide.

**8.** Procédé de la revendication 7, dans lequel l'étape de traitement thermique est réalisée à une température d'environ 70 °C à environ 80 °C.

**9.** Procédé de la revendication 7 ou la revendication 8, dans lequel l'étape de traitement acide est réalisée à un pH d'environ 3,9 à 4,4.

**10.** Procédé de l'une quelconque des revendications 7 à 9, dans lequel l'étape d'hydrolyse de protéine est réalisée

pendant une durée : (a) d'environ 15 minutes à environ 30 heures ; ou (b) d'environ 30 minutes à environ 2 heures.

**11.** Composition liquide stable présentant une viscosité inférieure à 4000 mPa·s (4000 cP) lorsqu'elle est mesurée par un rhéomètre comprenant :

(i) un ou une pluralité d'agents épaississants, dans lequel l'agent épaississant est choisi dans le groupe constitué de l'agar, de l'acide alginique, du carraghénane, de la gomme de guar, de la gomme adragante, de la gomme ghatti, de la cellulose microcristalline, de la carboxyméthylcellulose sodique, de la méthylcellulose, de l'hydroxypropylméthylcellulose, de l'hydroxypropylcellulose, de la méthyléthylcellulose, de la gomme karaya, de la gomme xanthane, de la gomme de caroube, de la gomme tara, de la gomme de graines de psyllium, de la gomme de graines de coing, d'une pectine, du furcellarane, de la gomme gellane, du konjac, de l'alginate de sodium et de toute combinaison de ceux-ci et dans lequel l'agent épaississant est présent en une quantité d'environ 3 % à environ 30 % en poids de la composition liquide stable ; et
(ii) un ingrédient à base de polysaccharide comprenant un matériau source à base de polysaccharide choisi dans le groupe constitué d'un extrait polysaccharidique de *Larix occidentalis,* d'un extrait polysaccharidique de *Larix laricina,* d'un extrait polysaccharidique d'acacia, d'un extrait polysaccharidique de *Larix decidua,* d'un extrait polysaccharidique de *Larix sibirica* et de toute combinaison de ceux-ci, dans lequel le matériau source à base de polysaccharide a été soumis à une étape d'hydrolyse de protéine ;

dans lequel l'ajout de la composition à un produit alimentaire sous forme de liquide aqueux ou de mélange liquide-solide aqueux augmente la viscosité dudit produit alimentaire.

**12.** Procédé permettant d'augmenter la viscosité d'un produit alimentaire sous forme de liquide aqueux ou de mélange liquide-solide aqueux, le procédé comprenant les étapes de :

(a) ajout au produit alimentaire d'une composition liquide stable présentant une viscosité inférieure à 4000 mPa·s (4000 cP) lorsqu'elle est mesurée par un rhéomètre comprenant :

(i) un ou une pluralité d'agents épaississants, dans lequel l'agent épaississant est choisi dans le groupe constitué de l'agar, de l'acide alginique, du carraghénane, de la gomme de guar, de la gomme adragante, de la gomme ghatti, de la cellulose microcristalline, de la carboxyméthylcellulose sodique, de la méthylcellulose, de l'hydroxypropylméthylcellulose, de l'hydroxypropylcellulose, de la méthyléthylcellulose, de la gomme karaya, de la gomme xanthane, de la gomme de caroube, de la gomme tara, de la gomme de graines de psyllium, de la gomme de graines de coing, d'une pectine, du furcellarane, de la gomme gellane, du konjac, de l'alginate de sodium et de toute combinaison de ceux-ci et dans lequel l'agent épaississant est présent en une quantité d'environ 3 % à environ 30 % en poids de la composition liquide stable ; et
(ii) un ingrédient à base de polysaccharide comprenant un matériau source à base de polysaccharide choisi dans le groupe constitué d'un extrait polysaccharidique de *Larix occidentalis,* d'un extrait polysaccharidique de *Larix laricina,* d'un extrait polysaccharidique d'acacia, d'un extrait polysaccharidique de *Larix decidua,* d'un extrait polysaccharidique de *Larix sibirica* et de toute combinaison de ceux-ci, dans lequel le matériau source à base de polysaccharide a été soumis à une étape d'hydrolyse de protéine ; et

(b) mélange du produit alimentaire et de la composition de manière à favoriser l'augmentation de la viscosité dudit produit alimentaire par la composition.

**13.** Procédé de production d'une composition liquide stable, comprenant les étapes de :

(i) fourniture d'un ingrédient à base de polysaccharide comprenant un matériau source à base de polysaccharide choisi dans le groupe constitué d'un extrait polysaccharidique de *Larix occidentalis,* d'un extrait polysaccharidique de *Larix laricina,* d'un extrait polysaccharidique d'acacia, d'un extrait polysaccharidique de *Larix decidua,* d'un extrait polysaccharidique de *Larix sibirica* et de toute combinaison de ceux-ci, dans lequel le matériau source à base de polysaccharide a été soumis à une étape d'hydrolyse de protéine ;
(ii) ajout d'un ou d'une pluralité d'agents épaississants à l'ingrédient à base de polysaccharide, dans lequel l'agent épaississant est choisi dans le groupe constitué de l'agar, de l'acide alginique, du carraghénane, de la gomme de guar, de la gomme adragante, de la gomme ghatti, de la cellulose microcristalline, de la carboxyméthylcellulose sodique, de la méthylcellulose, de l'hydroxypropylméthylcellulose, de l'hydroxypropylcellulose, de la méthyléthylcellulose, de la gomme karaya, de la gomme xanthane, de la gomme de caroube, de la gomme tara, de la gomme de graines de psyllium, de la gomme de graines de coing, d'une pectine, du furcellarane, de la

gomme gellane, du konjac, de l'alginate de sodium et de toute combinaison de ceux-ci et dans lequel l'agent épaississant est présent en une quantité d'environ 3 % à environ 30 % en poids de la composition liquide stable ; et

(iii) mélange du mélange de l'étape (ii) pour produire ainsi la composition liquide stable.

**14.** Composition liquide stable de la revendication 11, dans laquelle l'ingrédient à base de polysaccharide est celui selon l'une quelconque des revendications 1 à 6.

**15.** Procédé de la revendication 12 ou la revendication 13, dans lequel l'ingrédient à base de polysaccharide est celui selon l'une quelconque des revendications 1 à 6.

Polysaccharide-based solution, acids & preservatives
Bulk Aqueous
Sample 1 Bulk Retentate
Stage 1
Heat
Protein Removal
Sample 3 First Retentate
Sample 2 First Extract
First Xanthan addition
Stage 2
Protein removal
Second Xanthan addition
Sample 5 Second Retentate
Yes
Sample 4 Second Extract
Heat
Protein removal
Sample 7 Third Retentate
Sample 6 Third Extract
Stage 3
hold
Protein removal
Sample 8 Fourth Extract
Packing
Sample 9 Fourth Retentate

FIG. 1

Protein loss (%)
0%
-5%
-10%
-15%
-20%
-25%
Retentate 1
Retentate 2
Retentate 3

FIG. 2

| | TapWater (ml) | Saline Sol. (pumps) | ThickN (pumps) | Ohms |
|---|---|---|---|---|
| Full Apple | 85 | 3 | 0 | 184 |
| | 85 | 3 | 1 | 191 |
| | 85 | 3 | 2 | 187 |
| | 85 | 3 | 3 | 183 |
| Less Apple | 85 | 3 | 0 | 169 |
| | 85 | 3 | 1 | 168 |
| | 85 | 3 | 2 | 169 |
| | 85 | 3 | 3 | 168 |
| Original Saline | 75 | 5 | 0 | 170 |
| | 75 | 5 | 1 | 163 |
| | 75 | 5 | 2 | 165 |
| | 75 | 5 | 3 | 167 |

85ml Water/3 Pumps Full Apple Saline Solution
85ml Water/3 Pumps Reduced Apple Saline Solution
75ml Water/5 Pumps Original Saline Solution
Impedance (Ohms)
250
200
150
100
0
1
2
3
Pumps of Precise

FIG. 3

PM
1
2
3
4
5
6
7
8
PM
198 kDa
98 kDa
62 kDa
48 kDa
38 kDa
28 kDa
17 kDa
14 kDa
6 kDa
3 kDa
198 kDa
98 kDa
62 kDa
48 kDa
38 kDa
28 kDa
17 kDa
14 kDa
6 kDa
3 kDa

**FIG. 4**

T = Peptide from trypsin
466.7
441.7
415.7
399.2
507.7
705.3
680.6
T
T
T
11.256
11.145
11.547
12.442
13.298
13.697
14.934
15.214
15.934
16.327
18.659
8.761
8.517
8.112
7.683
Intensity
Time, min

FIG. 5

FIG. 6

Intensity
3.0e5
2.5e5
2.0e5
1.5e5
1.0e5
5.0e5
0.0e0
10
11
12
13
14
15
16
17
Time, min

FIG. 6 cont'd

XIC from 170904 TSC1 1 3xdil.wiff (sample 1) - 170904 TSC1..., Experiment 1, +TOF MS (350 - 1600): 680.640 +/- 0.015 Da
XIC from 170904 TSC1 1 3xdil.wiff (sample 1) - 170904 TSC1 1 3xdil, Experiment 1, +TOF MS (350 - 1600): 507.770 +/- 0.015 Da
XIC from 170904 TSC1 1 3xdil.wiff (sample 1) - 170904 TSC1 1 3xdil, Experiment 1, +TOF MS (350 - 1600): 399.220 +/- 0.015 Da
XIC from 170904 TSC1 1 3xdil.wiff (sample 1) - 170904 TSC1 1 3xdil, Experiment 1, +TOF MS (350 - 1600): 441.720 +/- 0.015 Da
XIC from 170904 TSC1 1 3xdil.wiff (sample 1) - 170904 TSC1 1 3xdil, Experiment 1, +TOF MS (350 - 1600): 415.780 +/- 0.015 Da
XIC from 170904 TSC1 1 3xdil.wiff (sample 1) - 170904 TSC1 1 3xdil, Experiment 1, +TOF MS (350 - 1600): 466.750 +/- 0.015 Da
XIC from 170904 TSC1 1 3xdil.wiff (sample 1) - 170904 TSC1 1 3xdil, Experiment 1, +TOF MS (350 - 1600): 705.360 +/- 0.015 Da
Intensity
1.0e5
9.0e4
8.0e4
7.0e4
6.0e4
5.0e4
4.0e4
3.0e4
2.0e4
1.0e4
0.0e0
10
11
12
13
14
15
16
17
Time, min

FIG. 7

XIC from 170904 TSC1 2 3xdil.wiff (sample 1) - 170904 TSC1 1 3xdil, Experiment 1, +TOF MS (350 - 1600): 680.640 +/- 0.015 Da
XIC from 170904 TSC1 2 3xdil.wiff (sample 1) - 170904 TSC1 ..., Experiment 1, +TOF MS (350 - 1600): 507.770 +/- 0.015 Da
XIC from 170904 TSC1 2 3xdil.wiff (sample 1) - 170904 TSC1 1 3xdil, Experiment 1, +TOF MS (350 - 1600): 399.220 +/- 0.015 Da
XIC from 170904 TSC1 2 3xdil.wiff (sample 1) - 170904 TSC1 1 3xdil, Experiment 1, +TOF MS (350 - 1600): 441.720 +/- 0.015 Da
XIC from 170904 TSC1 2 3xdil.wiff (sample 1) - 170904 TSC1 1 3xdil, Experiment 1, +TOF MS (350 - 1600): 415.780 +/- 0.015 Da
XIC from 170904 TSC1 2 3xdil.wiff (sample 1) - 170904 TSC1 1 3xdil, Experiment 1, +TOF MS (350 - 1600): 466.750 +/- 0.015 Da
XIC from 170904 TSC1 2 3xdil.wiff (sample 1) - 170904 TSC1 1 3xdil, Experiment 1, +TOF MS (350 - 1600): 705.360 +/- 0.015 Da
Intensity
1.2e5
1.1e5
1.0e5
9.0e4
8.0e4
7.0e4
6.0e4
5.0e4
4.0e4
3.0e4
2.0e4
1.0e4
0.0e0
10
11
12
13
14
15
16
17
Time, min

FIG. 7 cont'd

XIC from 170904 TSC1 3 3xdil.wiff (sample 1) - 170904 TSC1 1 ...il, Experiment 1, +TOF MS (350 - 1600): 680.640 +/- 0.015 Da
XIC from 170904 TSC1 3 3xdil.wiff (sample 1) - 170904 TSC... Experiment 1, +TOF MS (350 - 1600): 507.770 +/- 0.015 Da
XIC from 170904 TSC1 3 3xdil.wiff (sample 1) - 170904 TSC1 1 ...il, Experiment 1, +TOF MS (350 - 1600): 399.220 +/- 0.015 Da
XIC from 170904 TSC1 3 3xdil.wiff (sample 1) - 170904 TSC1 1 ...il, Experiment 1, +TOF MS (350 - 1600): 441.720 +/- 0.015 Da
XIC from 170904 TSC1 3 3xdil.wiff (sample 1) - 170904 TSC1 1 ...il, Experiment 1, +TOF MS (350 - 1600): 415.780 +/- 0.015 Da
XIC from 170904 TSC1 3 3xdil.wiff (sample 1) - 170904 TSC1 1 ...il, Experiment 1, +TOF MS (350 - 1600): 466.750 +/- 0.015 Da
XIC from 170904 TSC1 3 3xdil.wiff (sample 1) - 170904 TSC1 1 ...il, Experiment 1, +TOF MS (350 - 1600): 705.360 +/- 0.015 Da
Intensity
2.5e6
2.0e6
1.5e6
1.0e6
5.0e5
0.0e0
10
11
12
13
14
15
16
17
Time, min


FIG. 7 cont'd

XIC from 170904 TSC2 1 3xdil.wiff (sample 1) - 170904 TSC1... Experiment 1, +TOF MS (350 - 1600): 680.640 +/- 0.015 Da
XIC from 170904 TSC2 1 3xdil.wiff (sample 1) - 170904 TSC1 1 ...dil, Experiment 1, +TOF MS (350 - 1600): 507.770 +/- 0.015 Da
XIC from 170904 TSC2 1 3xdil.wiff (sample 1) - 170904 TSC1 1 ...dil, Experiment 1, +TOF MS (350 - 1600): 399.220 +/- 0.015 Da
XIC from 170904 TSC2 1 3xdil.wiff (sample 1) - 170904 TSC1 1 ...dil, Experiment 1, +TOF MS (350 - 1600): 441.720 +/- 0.015 Da
XIC from 170904 TSC2 1 3xdil.wiff (sample 1) - 170904 TSC1 1 ...dil, Experiment 1, +TOF MS (350 - 1600): 415.780 +/- 0.015 Da
XIC from 170904 TSC2 1 3xdil.wiff (sample 1) - 170904 TSC1 1 ...dil, Experiment 1, +TOF MS (350 - 1600): 466.750 +/- 0.015 Da
XIC from 170904 TSC2 1 3xdil.wiff (sample 1) - 170904 TSC1 1 ...dil, Experiment 1, +TOF MS (350 - 1600): 705.360 +/- 0.015 Da
Intensity
2.2e5
2.0e5
1.8e5
1.6e5
1.4e5
1.2e5
1.0e5
8.0e4
6.0e4
4.0e4
2.0e4
0.0e0
10
11
12
13
14
15
16
17
Time, min

FIG. 8

XIC from 170904 TSC2 2 3xdil.wiff (sample 1) - 170904 TSC1... Experiment 1, +TOF MS (350 - 1600): 580.640 +/- 0.015 Da
XIC from 170904 TSC2 2 3xdil.wiff (sample 1) - 170904 TSC1 1 ...il, Experiment 1, +TOF MS (350 - 1600): 507.770 +/- 0.015 Da
XIC from 170904 TSC2 2 3xdil.wiff (sample 1) - 170904 TSC1 1 ...il, Experiment 1, +TOF MS (350 - 1600): 399.220 +/- 0.015 Da
XIC from 170904 TSC2 2 3xdil.wiff (sample 1) - 170904 TSC1 1 ...il, Experiment 1, +TOF MS (350 - 1600): 441.720 +/- 0.015 Da
XIC from 170904 TSC2 2 3xdil.wiff (sample 1) - 170904 TSC1 1 ...il, Experiment 1, +TOF MS (350 - 1600): 415.760 +/- 0.015 Da
XIC from 170904 TSC2 2 3xdil.wiff (sample 1) - 170904 TSC1 1 ...il, Experiment 1, +TOF MS (350 - 1600): 456.750 +/- 0.015 Da
XIC from 170904 TSC2 2 3xdil.wiff (sample 1) - 170904 TSC1 1 ...il, Experiment 1, +TOF MS (350 - 1600): 705.360 +/- 0.015 Da
Intensity
4.0e5
3.5e5
3.0e5
2.5e5
2.0e5
1.5e5
1.0e5
5.0e4
0.0e0
10
11
12
13
14
15
16
17
Time, min


FIG. 8 cont'd

XIC from 170904 TSC2 3 3xdil.wiff (sample 1) - 170904 TSC2 3 ..., Experiment 1, +TOF MS (350 - 1600): 680.640 +/- 0.015 Da
XIC from 170904 TSC2 3 3xdil.wiff (sample 1) - 170904 TSC2... Experiment 1, +TOF MS (350 - 1600): 507.770 +/- 0.015 Da
XIC from 170904 TSC2 3 3xdil.wiff (sample 1) - 170904 TSC2 3 ..., Experiment 1, +TOF MS (350 - 1600): 399.220 +/- 0.015 Da
XIC from 170904 TSC2 3 3xdil.wiff (sample 1) - 170904 TSC2 3 ..., Experiment 1, +TOF MS (350 - 1600): 441.720 +/- 0.015 Da
XIC from 170904 TSC2 3 3xdil.wiff (sample 1) - 170904 TSC2 3 ..., Experiment 1, +TOF MS (350 - 1600): 415.780 +/- 0.015 Da
XIC from 170904 TSC2 3 3xdil.wiff (sample 1) - 170904 TSC2 3 ..., Experiment 1, +TOF MS (350 - 1600): 466.750 +/- 0.015 Da
XIC from 170904 TSC2 3 3xdil.wiff (sample 1) - 170904 TSC2 3 ..., Experiment 1, +TOF MS (350 - 1600): 705.360 +/- 0.015 Da
Intensity
5.0e6
4.5e6
4.0e6
3.5e6
3.0e6
2.5e6
2.0e6
1.5e6
1.0e6
5.0e5
0.0e0
10
11
12
13
14
15
16
17
Time, min

FIG. 8 cont'd

XIC from 170904 TSC3 1 3xdil.wiff (sample 1) - 170904 TSC1..., Experiment 1, +TOF MS (350 - 1600): 530.640 +/- 0.015 Da
XIC from 170904 TSC3 1 3xdil.wiff (sample 1) - 170904 TSC1 1 3xdil, Experiment 1, +TOF MS (350 - 1600): 507.770 +/- 0.015 Da
XIC from 170904 TSC3 1 3xdil.wiff (sample 1) - 170904 TSC1 1 3xdil, Experiment 1, +TOF MS (350 - 1600): 399.220 +/- 0.015 Da
XIC from 170904 TSC3 1 3xdil.wiff (sample 1) - 170904 TSC1 1 3xdil, Experiment 1, +TOF MS (350 - 1600): 441.720 +/- 0.015 Da
XIC from 170904 TSC3 1 3xdil.wiff (sample 1) - 170904 TSC1 1 3xdil, Experiment 1, +TOF MS (350 - 1600): 415.780 +/- 0.015 Da
XIC from 170904 TSC3 1 3xdil.wiff (sample 1) - 170904 TSC1 1 3xdil, Experiment 1, +TOF MS (350 - 1600): 466.750 +/- 0.015 Da
XIC from 170904 TSC3 1 3xdil.wiff (sample 1) - 170904 TSC1 1 3xdil, Experiment 1, +TOF MS (350 - 1600): 705.360 +/- 0.015 Da
Intensity
1.6e5
1.5e5
1.4e5
1.3e5
1.2e5
1.1e5
1.0e5
9.0e4
8.0e4
7.0e4
6.0e4
5.0e4
4.0e4
3.0e4
2.0e4
1.0e4
0.0e0
10
11
12
13
14
15
16
17
Time, min

FIG. 9

XIC from 170904 TSC3 2 3xdil.wiff (sample 1) - 170904 TSC1..., Experiment 1, +TOF MS (350 - 1600): 680.640 +/- 0.015 Da
XIC from 170904 TSC3 2 3xdil.wiff (sample 1) - 170904 TSC1 1 3xdil, Experiment 1, +TOF MS (350 - 1600): 507.770 +/- 0.015 Da
XIC from 170904 TSC3 2 3xdil.wiff (sample 1) - 170904 TSC1 1 3xdil, Experiment 1, +TOF MS (350 - 1600): 399.220 +/- 0.015 Da
XIC from 170904 TSC3 2 3xdil.wiff (sample 1) - 170904 TSC1 1 3xdil, Experiment 1, +TOF MS (350 - 1600): 441.720 +/- 0.015 Da
XIC from 170904 TSC3 2 3xdil.wiff (sample 1) - 170904 TSC1 1 3xdil, Experiment 1, +TOF MS (350 - 1600): 415.780 +/- 0.015 Da
XIC from 170904 TSC3 2 3xdil.wiff (sample 1) - 170904 TSC1 1 3xdil, Experiment 1, +TOF MS (350 - 1600): 468.750 +/- 0.015 Da
XIC from 170904 TSC3 2 3xdil.wiff (sample 1) - 170904 TSC1 1 3xdil, Experiment 1, +TOF MS (350 - 1600): 705.360 +/- 0.015 Da
Intensity
2.4e5
2.2e5
2.0e5
1.8e5
1.6e5
1.4e5
1.2e5
1.0e5
8.0e4
6.0e4
4.0e4
2.0e4
0.0e0
10
11
12
13
14
15
16
17
Time, min

FIG. 9 cont'd

XIC from 170904 TSC3 3 3xdil.wiff (sample 1) - 170904 TSC1... Experiment 1, +TOF MS (350 - 1600): 630.540 +/- 0.015 Da
XIC from 170904 TSC3 3 3xdil.wiff (sample 1) - 170904 TSC1 1 ...ll, Experiment 1, +TOF MS (350 - 1600): 507.770 +/- 0.015 Da
XIC from 170904 TSC3 3 3xdil.wiff (sample 1) - 170904 TSC1 1 ...ll, Experiment 1, +TOF MS (350 - 1600): 399.220 +/- 0.015 Da
XIC from 170904 TSC3 3 3xdil.wiff (sample 1) - 170904 TSC1 1 ...ll, Experiment 1, +TOF MS (350 - 1600): 441.720 +/- 0.015 Da
XIC from 170904 TSC3 3 3xdil.wiff (sample 1) - 170904 TSC1 1 ...ll, Experiment 1, +TOF MS (350 - 1600): 415.780 +/- 0.015 Da
XIC from 170904 TSC3 3 3xdil.wiff (sample 1) - 170904 TSC1 1 ...ll, Experiment 1, +TOF MS (350 - 1600): 466.750 +/- 0.015 Da
XIC from 170904 TSC3 3 3xdil.wiff (sample 1) - 170904 TSC1 1 ...ll, Experiment 1, +TOF MS (350 - 1600): 705.360 +/- 0.015 Da
Intensity
3.0e6
2.5e6
2.0e6
1.5e6
1.0e6
5.0e5
0.0e0
10
11
12
13
14
15
16
17
Time, min

FIG. 9 cont'd

XIC from 170904 TSC5 1 3xdil.wiff (sample 1) - 170904 TSC1... Experiment 1, +TOF MS (350 - 1600): 683.640 +/- 0.015 Da
XIC from 170904 TSC5 1 3xdil.wiff (sample 1) - 170904 TSC1 1 ...dil, Experiment 1, +TOF MS (350 - 1600): 507.770 +/- 0.015 Da
XIC from 170904 TSC5 1 3xdil.wiff (sample 1) - 170904 TSC1 1 ...dil, Experiment 1, +TOF MS (350 - 1600): 399.220 +/- 0.015 Da
XIC from 170904 TSC5 1 3xdil.wiff (sample 1) - 170904 TSC1 1 ...dil, Experiment 1, +TOF MS (350 - 1600): 441.720 +/- 0.015 Da
XIC from 170904 TSC5 1 3xdil.wiff (sample 1) - 170904 TSC1 1 ...dil, Experiment 1, +TOF MS (350 - 1600): 415.780 +/- 0.015 Da
XIC from 170904 TSC5 1 3xdil.wiff (sample 1) - 170904 TSC1 1 ...dil, Experiment 1, +TOF MS (350 - 1600): 466.750 +/- 0.015 Da
XIC from 170904 TSC5 1 3xdil.wiff (sample 1) - 170904 TSC1 1 ...dil, Experiment 1, +TOF MS (350 - 1600): 705.360 +/- 0.015 Da
Intensity
1.8e5
1.6e5
1.4e5
1.2e5
1.0e5
8.0e4
6.0e4
4.0e4
2.0e4
0.0e0
10
11
12
13
14
15
16
17
Time, min

FIG. 10

XIC from 170904 TSC5 2 3xdil.wiff (sample 1) - 170904 TSC1... Experiment 1, +TOF MS (350 - 1600): 680.640 +/- 0.015 Da
XIC from 170904 TSC5 2 3xdil.wiff (sample 1) - 170904 TSC1 1 ...dil, Experiment 1, +TOF MS (350 - 1600): 507.770 +/- 0.015 Da
XIC from 170904 TSC5 2 3xdil.wiff (sample 1) - 170904 TSC1 1 ...dil, Experiment 1, +TOF MS (350 - 1600): 399.220 +/- 0.015 Da
XIC from 170904 TSC5 2 3xdil.wiff (sample 1) - 170904 TSC1 1 ...dil, Experiment 1, +TOF MS (350 - 1600): 441.720 +/- 0.015 Da
XIC from 170904 TSC5 2 3xdil.wiff (sample 1) - 170904 TSC1 1 ...dil, Experiment 1, +TOF MS (350 - 1600): 415.780 +/- 0.015 Da
XIC from 170904 TSC5 2 3xdil.wiff (sample 1) - 170904 TSC1 1 ...dil, Experiment 1, +TOF MS (350 - 1600): 466.750 +/- 0.015 Da
XIC from 170904 TSC5 2 3xdil.wiff (sample 1) - 170904 TSC1 1 ...dil, Experiment 1, +TOF MS (350 - 1600): 705.360 +/- 0.015 Da
Intensity
3.0e5
2.5e5
2.0e5
1.5e5
1.0e5
5.0e4
0.0e0
10
11
12
13
14
15
16
17
Time, min

FIG. 10 cont'd

XIC from 170904 TSC8 2 3xdil.wiff (sample 1) - 170904 TSC1..., Experiment 1, +TOF MS (350 - 1600): 680.640 +/- 0.015 Da
XIC from 170904 TSC8 2 3xdil.wiff (sample 1) - 170904 TSC1 1 3xdil, Experiment 1, +TOF MS (350 - 1600): 507.770 +/- 0.015 Da
XIC from 170904 TSC8 2 3xdil.wiff (sample 1) - 170904 TSC1 1 3xdil, Experiment 1, +TOF MS (350 - 1600): 399.220 +/- 0.015 Da
XIC from 170904 TSC8 2 3xdil.wiff (sample 1) - 170904 TSC1 1 3xdil, Experiment 1, +TOF MS (350 - 1600): 441.720 +/- 0.015 Da
XIC from 170904 TSC8 2 3xdil.wiff (sample 1) - 170904 TSC1 1 3xdil, Experiment 1, +TOF MS (350 - 1600): 415.780 +/- 0.015 Da
XIC from 170904 TSC8 2 3xdil.wiff (sample 1) - 170904 TSC1 1 3xdil, Experiment 1, +TOF MS (350 - 1600): 466.750 +/- 0.015 Da
XIC from 170904 TSC8 2 3xdil.wiff (sample 1) - 170904 TSC1 1 3xdil, Experiment 1, +TOF MS (350 - 1600): 705.360 +/- 0.015 Da
Intensity
1.8e5
1.6e5
1.4e5
1.2e5
1.0e5
8.0e4
6.0e4
4.0e4
2.0e4
0.0e0
10
11
12
13
14
15
16
17
Time, min

**FIG. 11**

XIC from 170904 TSC8 3 3xdil.wiff (sample 1) - 170904 TSC1... Experiment 1, +TOF MS (350 - 1600): 680.640 +/- 0.015 Da
XIC from 170904 TSC8 3 3xdil.wiff (sample 1) - 170904 TSC1 1 ...dil, Experiment 1, +TOF MS (350 - 1600): 507.770 +/- 0.015 Da
XIC from 170904 TSC8 3 3xdil.wiff (sample 1) - 170904 TSC1 1 ...dil, Experiment 1, +TOF MS (350 - 1600): 399.220 +/- 0.015 Da
XIC from 170904 TSC8 3 3xdil.wiff (sample 1) - 170904 TSC1 1 ...dil, Experiment 1, +TOF MS (350 - 1600): 441.720 +/- 0.015 Da
XIC from 170904 TSC8 3 3xdil.wiff (sample 1) - 170904 TSC1 1 ...dil, Experiment 1, +TOF MS (350 - 1600): 415.780 +/- 0.015 Da
XIC from 170904 TSC8 3 3xdil.wiff (sample 1) - 170904 TSC1 1 ...dil, Experiment 1, +TOF MS (350 - 1600): 466.750 +/- 0.015 Da
XIC from 170904 TSC8 3 3xdil.wiff (sample 1) - 170904 TSC1 1 ...dil, Experiment 1, +TOF MS (350 - 1600): 705.360 +/- 0.015 Da
Intensity
2.2e6
2.0e6
1.8e6
1.6e6
1.4e6
1.2e6
1.0e6
8.0e5
6.0e5
4.0e5
2.0e5
0.0e0
10
11
12
13
14
15
16
17
Time, min


FIG. 11 cont'd

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006053761 A
- US 20040197456 A, Holahan
- AU 2017050966 W
- US 6455090 B, Uzahashi


### Non-patent literature cited in the description


- The EC number refers to Enzyme Nomenclature. Academic Press, 1992
- Handbook of Proteolytic Enzymes. Academic Press, 1998
- **PETERSEN et al.** *Determination of the Degree of Hydrolysis (DH) based on OPA Reaction*, December 1995
- **FRISTER et al.** OPA method modified by use of N,N-dimethyl-2-mercaptoethylammonium chloride as thiol component. *Fresenius J. Anal. Chem.*, 1988, vol. 330, 631
- AOAC 968.06-1969, Protein (Crude) in animal feed. Dumas method. **JONES, D. B.** Factors for converting percentages of nitrogen in foods and fees into percentages of proteins. US Department of Agriculture, 1931